(19) Europäisches Patentamt
European Patent Office
Office européen des brevets

(11) Publication number: 0 462 224 B1

# (12) EUROPEAN PATENT SPECIFICATION

(45) Date of publication of patent specification: 31.08.94 (51) Int. Cl.[5]: C12Q 1/68

(21) Application number: 90905769.7

(22) Date of filing: 09.03.90

(86) International application number:
PCT/US90/01316

(87) International publication number:
WO 90/10718 (20.09.90 90/22)

## (54) SEOUENCING NUCLEIC ACIDS USING CHEMILUMINESCENT DETECTION.

(30) Priority: 10.03.89 US 322249

(43) Date of publication of application:
27.12.91 Bulletin 91/52

(45) Publication of the grant of the patent:
31.08.94 Bulletin 94/35

(84) Designated Contracting States:
CH DE FR GB LI

(56) References cited:
EP-A- 0 070 687
EP-A- 0 131 830
EP-A- 0 185 547
EP-A- 0 210 449
EP-A- 0 284 660
WO-A-89/02932
DE-A- 2 915 082

Dialog Information Services, Fi8le 154, Medline 83-90, MEDLINE accession no. 89345075, Beck S et al.: "Chemiluminescent detection of DNA: application for DNA sequencing an hybridization", Nucleic Acids Res (ENGLAND) Jul 11 1989, 17 (13) p. 5115-5123

(73) Proprietor: MILLIPORE CORPORATION
80 Ashby Road
Bedford Massachusetts 01730 (US)

(72) Inventor: BECK, Stephan
128 Maple Street
Lexington, MA 02173 (US)
Inventor: COULL, James, M.
57 Parker Street
Acton, MA 01720 (US)
Inventor: KOESTER, Hubert
1640 Monument Street
Concord, MA 01742 (US)
Inventor: O'KEEFFE, Thomas, J.
14 Karen Road
Windham, NH 03087 (US)

(74) Representative: Holdcroft, James Gerald, Dr. et al
Graham Watt & Co.,
Riverhead
Sevenoaks, Kent TN13 2BN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

### Background of the Invention

The genetic information of all living organisms is imprinted in deoxyribonucleic acid (DNA) and ribonucleic acid (RNA). DNA is made of four chemical building blocks, the nucleotides, deoxyadenosine-5'-monophosphate (A), deoxyguanosine-5'-monophosphate (G), deoxycytosine-5'-monophosphate (C) and deoxythymidin-5'-monophosphate (T), which are linearly aligned to yield individually different and specific sequences. The elucidation of the DNA sequence of individual genes or complete genomes plays a key role in molecular biology. The building blocks of RNA can be similarily aligned to yield specific sequences.

Two basic methods for DNA sequencing have been developed: A method of chemical degradation, which uses modifying reagents that are nucleobase specific (Maxam, A.M. and Gilbert, W., Proc. Natl. Acad. Sci. USA 74:560 (1977)) and an enzymatic method which uses DNA polymerases and nucleobase-specific, chain-terminating dideoxynucleoside triphosphates (Sanger, F. et al., Proc. Natl. Acad. Sci. USA 74:5463 (1977)). Due to the importance of DNA sequencing and to the goal of obtaining the DNA sequence of entire genomes, such as the human genome (e.g., Hood, L.E. and Smith, L.M., Biotechnology 5:933 (1987)), many new DNA cloning, mapping and sequencing techniques have been developed (e.g., Methods in Enzymology (1987), 155, Wu., R., editor, Academic Press, New York; Saluz, H.P. and Jost, J.P., A Laboratory Guide to Genomic Sequencing, Birkhauser-Verlag (1987), Basel, Boston; Wada, A. Nature 325:771 (1987)).

In general, DNA sequencing procedures consist of several main steps:

1) Selecting a DNA clone from a sequencing library (Sanger, F. et al., J. Mol. Biol., 162:729; (1982) Deininger, P.L., Anal. Biochem. 129:216 (1983); Heinikoff, S., Gene 28:351 (1984)).

2) Subjecting the selected DNA clone to base-specific reactions (Maxam, A.M. and Gilbert, W., Proc. Natl. Acad. Sci. USA 74:560 (1977); Sanger, F. et al., Proc. Natl. Acad. Sci. USA 74:5463 (1977)).

3) Separating the DNA reaction products according to size by denaturing polyacrylamide gel electrophoresis (PAGE).

4) Detecting the separated DNA band pattern after or during denaturing PAGE by any of a variety of techniques depending on whether the DNA has been labeled with radioisotopes (e.g., $^{32}P$, $^{35}S$) or non-radioactive marker molecules (e.g. fluorophores, colorimetric staining). Connell et al., BioTechniques 5:342 (1987); Prober, J.M. et al., Science 238:336 (1987); Smith, L.M. et al., Nature 321:674 (1986); Ansorge, W. et al., Nucleic Acids Res. 15:4593 (1987); Kambara, H. et al., BioTechniques 6:816 (1988); Beck, S., Anal. Biochem. 164:514 (1987)).

5) Reading the separated DNA bands as a sequence of A, G, C and T with the aid of sonic digitizers and computers (Bankier, A.T. and Barrell, B.G., Techniques in the Life Sciences, volume b5, Flavell, R.A. (editor), Elsevier, Ireland, pp. 1-34 (1983); Staden, R., Nucleic Acids Res. 12:499 (1984); Elder, J. et al., Nucleic Acids Res. 14:417 (1985); Connell et al., BioTechniques 5:342 (1987); Prober, J.M. et al., Science 238:336 (1987); Smith, L.M. et al., Nature 321:674 (1986); Ansorge, W. et al., Nucleic Acids Res. 15:4593 (1987); Kambara, H. et al., BioTechniques 6:816 (1988)).

Some of the steps of different sequencing processes are summarized below:

1) DNA sequencing using polymeric carriers:

In this approach, the DNA to be sequenced is immobilized on a solid support (e.g., CCS-paper, DEAE-paper, avidin-agarose) (See Chuvpilo, S.A. and Kravchenco, V.V., Biorg. Khim 9:1694 (1983); Rosenthal, A. et al., Nucleic Acids Res. 13:1173 (1985); Rosenthal, A. et al., Gene 42:1 (1986); Rosenthal, A. et al., European Patent Application 0171072; Stahl, S. et al., Nucleic Acids Res. 16:3038 (1988). The advantage of immobilization on a solid phase is that the various reagents used in the sequencing reactions can easily be applied and quantitatively removed resulting in faster, cleaner and more efficient reaction cycles. In addition, the entire operation is more convenient and easier to automate. Both the chemical degradation and the enzymatic DNA sequencing methods can be performed on polymeric supports. Solid supports also offer the possibility of processing the sequencing reactions of several samples simultaneously.

2) Multiplex DNA Sequencing:

In standard DNA sequencing procedures, each sample of DNA to be sequenced must be processed individually through the five steps outlined above. The "throughput" is limited because many steps in a sequencing procedure must be repeated numerous times. Multiplex DNA sequencing provides a solution to this problem and is currently the most promising high throughput DNA sequencing technique (Church, G. and Kieffer-Higgins, S., Science 240:185 (1988)). Multiplex DNA sequencing reduces the number of repetitive steps by pooling (multiplexing) different DNA samples to be sequenced. The DNA is pooled in the form of plasmid-bearing colonies obtained from the initial recombinant DNA libraries. The key

element of the multiplex approach is the design of a set of sequencing vectors each of which differ only in two 20 nucleotide long "tag" sequences. This allows differentiation and detection of individual sequencing patterns by employing labeled "tag" oligodeoxynucleotides which are complementary to the corresponding marker sequence.

Genomic DNA fragments (which may be obtained by "shotgun" selection) are cloned in individual muliplex sequencing vectors and amplified in a pool. The pooled samples are then subject to base-specific reactions and the resulting reaction products are separated according to size by denaturing PAGE. All sequencing bands of a given pool of DNA samples are transferred (blotted) onto a membrane and immobilized by UV-crosslinking. Multiple reprobing (hybridization) of the membrane with the set of specific tag-oligodeoxynucleotides allows one to visualize the individual sequencing band pattern (for example, in the case of a radioactive label, by autoradiography). Thus, each multiplex reaction and electrophoretic separation yields a quantity of data which exceeds that of a standard sequencing run by the number of probing cycles employed. For example, when twenty individual multiplex vectors are used, forty probing cycles can be performed to obtain the sequence of each of the two DNA strands integrated into the 20 vectors. This requires a 40-fold higher sensitivity of detection than standard DNA sequencing, assuming that each lane of the sequencing gel contains the same amount of sequenced DNA in both methods. Currently, sufficient sensitivity can only be obtained when more material is applied to PAGE and/or when $^{32}$P-labeled tag-oligodeoxynucleotides are used.

3) Direct blotting electrophoresis (DBE):

DBE combines the electrophoretic separation and the blotting of DNA sequencing bands onto an immobilizing membrane (Pohl, F.M., U.S. Patent 4,631,122 and 4,631,120; Beck, S. and Pohl, F.M., EMBO J. 3:2905 (1984); Pohl, F.M. and Beck, S., "Direct Transfer Electrophoresis Used for DNA Sequencing" in Methods of Enzymology, Wu, R., editor, Academic Press 155:250, New York, (1987)). In contrast to the sequencing "ladders" obtained by standard denaturing PAGE, the band spacing with DBE is constant over a wide range of molecular weights (50-550 nucleotides for a 6% polyacrylamide gel). Band spacing can be varied by the speed with which the membrane is moved perpendicular to the electrophoresis gel; thus, by comparison to standard sequencing gels resolution is increased and the length of the electrophoresis gel is reduced. In multiplex DNA sequencing, DBE advantageously provides the necessary separation of the sequencing reaction products and a method for directly transferring the sequencing ladder onto a membrane. The sequence reaction products can then be permanently immobilized onto the membrane by UV-crosslinking and the information read by multiple probing. In the case of radioactively labeled DNA fragments, the sequence pattern of each probing cycle is visualized by autoradiography.

4) Polymerase Chain Reaction (PCR):

PCR allows a target DNA molecule to be amplified in an in vitro system rather than in a vector/host system in vivo. Two short oligodeoxynucleotide fragments are used as primers. The primers are hybridized to the distal portions of the minus- and plus-strands of the DNA fragment to be amplified and thermostable DNA polymerases catalyze synthesis of the complementary DNA strand (Saiki, R.K. et al., Science 239:487 (1988); Erlich, H.A. et al., European Patent Application 0258017; Mullis, K.B., European Patent Application 0201184). In addition to the convenience of eliminating the cloning steps to amplify a genomic DNA fragment to a level sufficient to allow DNA sequencing, a significant advantage of the PCR method results from the specific hybridization of the two primers to the target DNA. A DNA fragment can be amplified out of a mixture of DNA molecules even if it is present in a very small amount, as long as the distal sequences to which two primers can specifically hybridize are known. Moreover, by labeling either one of the two primers and by employing dideoxynucleoside triphosphates as specific terminators for the DNA polymerase chain reaction during a later stage of the amplification, the sequence of the target DNA can be obtained (Innis, M.A. et al., Proc. Natl. Acad. Sci. USA 85:9436 (1988); Saiki, R.K. et al., Science 239:487 (1988); Erlich, H.A. et al., European Patent Application 0258017; Mullis, K.B., European Patent Application 0201184). By using dideoxy multiplex DNA sequencing, the primer(s) need not be labeled. Visualization is accomplished by employing "tag" probes.

5) Non-radioactive detection for DNA sequencing:

Detection methods which are based upon non-radioactive molecules are advantageous because radioactive isotopes are costly, require special waste disposal and pose serious health risks. This is of particular importance in DNA sequencing because sequencing demands the use of a significant amount of radioactivity. Extensive DNA sequencing projects particularly are hampered by considerable safety and waste disposal problems. Additional disadvantages of radioactive labeling in DNA sequencing are the relatively short half life of $^{32}$P and $^{35}$S radioisotopes and the likelihood of damaging the DNA by radiolysis. These factors reduce the time in which the labeled primers/probes and deoxynucleoside

triphosphates can be used. Hence, non-radioactive detection in DNA sequencing is virtually a necessary prerequisite for large scale DNA sequencing projects.

So far, two different approaches to non-radioactive DNA sequencing have been reported. One method is based upon fluorescence. The synthetic sequencing primer (Sinha, N.D. et al., Nucleic Acids Res. 12:4539 (1984)) is specifically labeled at the 5' end via a primary amino group (Coull, J. et al., Tetrahedron Lett. 27:3991 (1986); Agrawal, S. et al., Nucleic Acids Res. 14:6227 (1986)) with a fluorophore such as fluorescein or rhodamine (Smith, L.M., et al., Nucleic Acids Res. 13:2399 (1985); Smith, L.M., et al., Nature 321: 674 (1986); Hood, L.E., et al., German Patent Application DE 3501306; Fung, S., et al., European Patent Application 0233053; Ansorge, W., et al., Nucleic Acids Res. 15:4593 (1987)). Alternatively, the fluorophore is introduced onto base-modified deoxyuridine moieties during chemical oligodeoxynucleotide synthesis (Brumbaugh, J.A., et al., Proc. Natl. Acad. Sci. USA 85:5610 (1988); Brumbaugh, J.A., et al., European Patent Application 0157280)). The latter approach allows the specific introduction of more than one fluorescent label into the sequencing primer and thus enhances the signal and sensitivity. In another approach, the terminating dideoxyribonucleoside triphosphates rather than the primer are labeled fluorescently (Prober, J.M., Science 238:336 (1987); Hobbs, F.W., et al., European Patent Application 0251786)). This approach is attractive because, when only the specific terminating nucleotides are labeled, all nonspecific chain terminations which occur during the sequencing reactions remain undetectable. This gives a lower background and better signal to noise ratio. When four different fluorescent labels are used to discriminate between the four bases A, G, C and T, the four separate sequencing reactions can be separated simultaneously in one lane during denaturing PAGE. With laser excitation and four dye-specific filters the lanes of an electrophoretic gel can be horizontally scanned on-line and the sequence data can be processed by a computer for further data refinement. This four-dye-one-lane approach (Smith, L.M., et al., Nature 321:674 (1986); Prober, J.M., et al., Science 238:336 (1987)) increases the throughput of a sequencing gel by a factor of four. Automated DNA sequencers for the primer or terminator labeling systems are commercially available from Applied Biosystems and DuPont de Nemours. The technical realization of these two approaches is more complex and expensive and it requires sophisticated data manipulation to read the DNA sequence with an appropriate level of error compared to the one-dye-four-lane approach (Ansorge, W., et al., Nucleic Acids Res. 15:4593 (1987)).

Although successfully employed for labeling primers/probes and terminators, and commercialized with semi-automatic instrumentation and appropriate fluorescent reagents, fluorescent labeling has several drawbacks. First, all systems developed so far do not produce a sequence band pattern that is devoid of electronic manipulation. An electronically non-filtered image of the primary sequencing data is essential for obtaining accurate sequence results. Despite numerous improvements, the chemical sequencing method of Maxam and Gilbert and the enzymatic sequencing method of Sanger are still not error-free and sometimes produce ambiguous results. Therefore, the evaluation of a direct image of the primary sequencing data by an expert is often necessary in order to discriminate unequivocally between reliable and ambiguous results. It is also important to store the image (i.e. x-ray film or photograph) so that it remains available for later comparisons or to better assess ambiguous sequence areas.

Secondly, fluorescent labeling does not achieve the overall sensitivity that is obtained with radioactive labeling. Thus, fluorescent detection can not be used for multiplex DNA sequencing which is currently the most promising large scale DNA sequencing technique available. Moreover, multiple reprobing of membrane-immobilized DNA fragments using fluorescently labeled oligodeoxynucleotides is not feasable due to the self-fluorescence of all currently available membranes. Lastly, fluorescently labeled materials are subject to photobleaching.

The second non-radioactive detection method entails colorimetric detection and DBE (Beck, S., Anal. Biochem. 164:514 (1987); Richterich, P., et al., BioTechniques, 7:52-59 (1989)). Specific colorimetric labeling of the sequencing primer is accomplished by a multistep process. First, the synthetic oligodeoxynucleotide primer is labeled at its 5' end with biotin (Coull, J. et al., Tetrahedron Lett. 27:3991 (1986); Agrawal, S., et al., Nucleic Acids. Res. 14:6227 (1986)). The oligonucleotides obtained by chain termination in the reaction mixtures are separated according to size by denaturing PAGE. The separated oligonucleotides are either transferred onto a membrane by standard techniques or are blotted directly onto a membrane by DBE. After covalently binding the oligonucleotides onto the membrane by UV crosslinking, the biotinylated fragments are visualized using a biotinylated enzyme, such as alkaline phosphatase, which can be anchored to the biotinylated DNA fragments via streptavidin which has an extremely high binding affinity for biotin. In addition, phosphataseavidin/streptavidin conjugates can be employed for this purpose. After labeling of all sequencing reaction products with alkaline phosphatase, a colorimetric reaction on a color producing substrate (e.g., BCIP: 5-bromo-4-chloro-3-indolyl phosphate; NBT: nitroblue tetrazolium) can develop the sequencing band pattern as colored bands.

Nucleic acids can be labeled with biotin chemically (as mentioned above) or enzymatically (Langer, P.R., et al., Proc. Natl. Acad. Sci. USA 78:6633 (1981); Ward, D.C, et al., US Patent 4,711,955; Riley, L.K., et al., DNA 5:333 (1986)). The biotin/avidin system has been widely used to label a variety of biomolecules, including nucleic acids for hybridization experiments (Wilchek, M., et al., Anal. Biochem. 171:1 (1988); Leary, J.J., et al., Proc. Natl. Acad. Sci. USA 80:4045 (1983); Chu, B.C.F., et al., DNA 4:327 (1985); Koch, J., et al., Nucleic Acids Res. 14:7133 (1986); Kourilsky, P., et al., US Patent 4,581,333; Ward, D.C., et al., US Patent 4,687,732)).

A permanent image of the primary sequencing data can be obtained using colorimetric labeling. There is, however, currently no efficient quantitative destaining method available, which does not affect the membrane-immobilized sequence information. Thus, multiplex DNA sequencing is not feasible using colorimetric labeling methods. Furthermore, the sensitivity which can be achieved may not be sufficient for multiplex DNA sequencing.

In spite of the number of available methods for determining nucleic acid sequences, there is a need for a detection system which eliminates the problems encountered by the use of radioactive, fluorescent or colorimetric labeling.

According to one aspect of the present invention, there is provided a method of detecting a nucleic acid chemiluminescense, comprising the steps of:

a. providing a nucleic acid which is linked to a compound which can generate chemiluminescence wherein the compound is an enzyme which catalyzes a chemiluminogenic substrate to emit light;
b. contacting the nucleic acid with a substituted 1,2-dioxetane chemiluminogenic substrate, thereby causing the compound to generate chemiluminescense; and
c. detecting the chemiluminescense, wherein light emitted is indicative of the presence and amount of the nucleic acid.

According to another aspect of the invention, there is provided a method of sequencing DNA, comprising the steps of:

a. providing a nucleic acid primer complementary to region of DNA to be sequenced, the primer being modified for attaching a compound which can generate chemiluminescense thereto;
b. hybridizing the primer to the DNA to be sequenced;
c. extending the primer with deoxyribonucleoside triphosphates using the DNA to be sequenced as a template and terminating the extension reactions with dideoxyribonucleoside triphosphates to produce DNA molecules whose lengths depend on the point of termination from the primer;
d. separating the DNA molecules generated by each of the extension reactions according to size;
e. affixing the separated DNA molecules to a solid support;
f. attaching a compound that can generate chemiluminescense to the separated molecules at the modified primer, wherein the compound which can generate chemiluminescence is an enzyme selected from the group consisting of alkaline phosphatase, acid phosphatase, peroxidase, galactosidase, glucose oxidase, luciferase, aryl esterase, sulfatase, urease, acetylcholinesterase, chymotrypsin, trypsin, aminopeptidase and lipase;
g. causing the compound to generate chemiluminescense by contacting the enzyme with a substituted 1,2-dioxetane chemiluminogenic substrate;
h. detecting the chemiluminescence, wherein light emitted is indicative of the presence and amount of nucleic acid, to obtain a pattern of the separated DNA molecules according to size; and
i. determining nucleotide sequence based upon the pattern of separated DNA molecules.

Further methods of sequencing DNA are defined in claims 8 and 9 appended hereto.

Still further according to the present invention, there is provided a method of hybridizing DNA, comprising the steps of:

a. providing nucleic acid to be probed which is affixed to a solid support;
b. contacting the nucleic acid with a probe complementary to a region of DNA to be hybridized, the probe being linked to a compound which can generate chemiluminescence wherein the compound is an enzyme which catalyzes a chemiluminogenic substrate to emit light;
c. hybridizing the probe to the DNA to be sequenced;
d. contacting the nucleic acid with a substituted 1,2-dioxetane chemiluminogenic substrate, thereby causing the compound to generate chemiluminescence; and
e. detecting the chemiluminescence wherein the light emitted is indicative of the presence and amount of the hybridized DNA.

This invention pertains to the use of chemiluminescence to detect nucleic acid molecules and to methods of sequencing nucleic acids using chemiluminescent detection. The sequencing methods are fast and highly sensitive for determining nucleic acid (DNA/RNA) sequences and they eliminate the use of

radioactive, fluorescent and colorimetric visualization methods.

According to the method of this invention, a nucleic acid is provided that is linked covalently or non-covalently to a compound that can generate chemiluminescence. The nucleic acid is provided as the result of a DNA sequencing procedure, for example.

The compound that can generate chemiluminescence can be an enzyme that catalyzes a chemiluminogenic substrate to emit light. The compound, however, can be one which itself can be induced to emit light.

To detect the nucleic acid, chemiluminescence is generated by an appropriate means. When the nucleic acid is linked to an enzyme, it is contacted with a chemiluminogenic substrate. The enzyme then triggers the substrate to emit light. Alternatively, if the chemiluminescence generating compound itself is chemiluminogenic, it is triggered to emit light by an appropriate stimulus (for example, pH, temperature and chemical reactions, including enzymatic reactions). In one embodiment, the step of inducing the chemiluminescence reaction can be performed in a homogeneous phase. The light emitted by the chemiluminescent reaction is detected and is indicative of the presence and the amount of the nucleic acid.

In the preferred embodiment, the nucleic acid is linked to an enzyme and the chemiluminogenic substrate for the enzyme is a stable, enzymatically triggerable substituted 1,2-dioxetane. In a particularly preferred embodiment, an enhancer molecule is used in connection with the chemiluminogenic substrate to amplify the intensity of light emitted. Preferably, the enhancer molecule is a fluorophore which itself is excited by light energy generated in the chemiluminescent reaction and emits light in its excited state. The amplified signal is detected.

The compound which is capable of generating chemiluminescence can be incorporated into the nucleic acid by covalently or non-covalently attaching it to one or more sites on the nucleic acid. For example, the compound can be attached to the 5' and/or 3' terminus, to one or more of the nucleobases or to one or more sites on the phosphodiester backbone. In addition, the compound can be attached to the nucleic acid via organic linker molecules, biotin/avidin (streptavidin) bridges, and bispecific antibodies.

The methods for detecting nucleic acids by chemiluminescence can be used in a number of nucleic acid sequencing techniques such as the chemical degradative method of Maxam and Gilbert, the enzymatic/primer extension method of Sanger and multiplex DNA sequencing or multiplexing techniques based upon either of the two sequencing chemistries. The methods can also be used in nucleic acid hybridization reactions. The method of attaching the compound to the nucleic acid has to be in compliance with the sequencing method employed.

In nucleic acid sequencing procedures, nucleic acids can be linked to a compound which can generate chemiluminescence at various stages of the sequencing process. When the nucleic acid is sequenced using a primer or chain terminator, the chemiluminescence generating compound can be attached to the nucleic acid after the products of the sequencing reaction are separated using known separation techniques. The chemiluminescence generating compound can be linked to the products of a sequencing reaction at one or more sites on the nucleic acid as described above. When the nucleic acid sequences are detected using a hybridization probe, the chemiluminescence generating compound can be linked at one or more sites on the probe.

The method of detecting nucleic acids by chemiluminescence has a number of advantages over the other methods of detection. The labeled nucleic acids obtained by using, e.g. primers, probes, and terminators have a long shelf-life. The hazards associated with radioactive material are eliminated. Permanent images of the sequencing data can be obtained easily on x-ray or instant films. High sensitivity can be achieved by various means of amplification such as by intermolecular energy transfer between a chemiluminescent compound and an enhancer molecule. Further, the detection system of this invention is highly sensitive and film exposure times are decreased. Moreover, with currently available charge coupled device cameras, the sequencing image can be directly obtained, thus avoiding the film step. The methods of this invention can be made part of an on-line detection system using direct blotting electrophoresis (DBE), as well as generate a permanent image of the sequencing band pattern. Additionally, the detection methods can be applied to multiplex DNA sequencing, as well as polymerase chain reaction (PCR) techniques, solid phase sequencing and DBE.

Brief Description of the Drawings

Figure 1 shows examples of stable and selectively triggerable 1,2-dioxetanes. Modified from Schaap, A.P., J. Bioluminescence and Chemiluminescence; DeLucam, M.A. and McElroy, W.D. (eds) 1981 Academic Press, New York.

Figure 2 shows a schematic diagram for the chemiluminescent detection of immobilized DNA fragments using an enzyme catalyzed reaction.

Figure 3 shows a dot blot after hybridization with biotinylated oligomer Bio15M13 (see Example 1,2) after chemiluminescent detection (see Example 3). From top to bottom 50, 10, 2, 0.4, 0.08, 0.0 femtomoles of single stranded M13 DNA were applied to a strip of nylon membrane (Nytran™). The strip was hybridized and processed as described in Example 2. [1] shows the result of the first probing after 1 sec (a), 5 sec (b), 10 sec (c) and 45 sec (d) exposure time to a Fuji x-ray film. (e) shows the same membrane after the probe had been removed (300 sec exposure time). [2], [20] and [40] show the result for the corresponding probing cycles number [2], [20] and [40] (same exposure times as for probing cycle number 1).

Figure 4 shows the chemiluminescent detection of the M13mp8 DNA sequence band pattern after DBE onto a nylon membrane (Nytran™). After processing the membrane as described in Example 4, the DNA sequence band pattern was detected (as described in Example 3) in a luminometer for 1 sec (a), 5 sec (b), 10 sec (c) and 45 sec (d) by exposure to a Polaroid 57 black and white film.

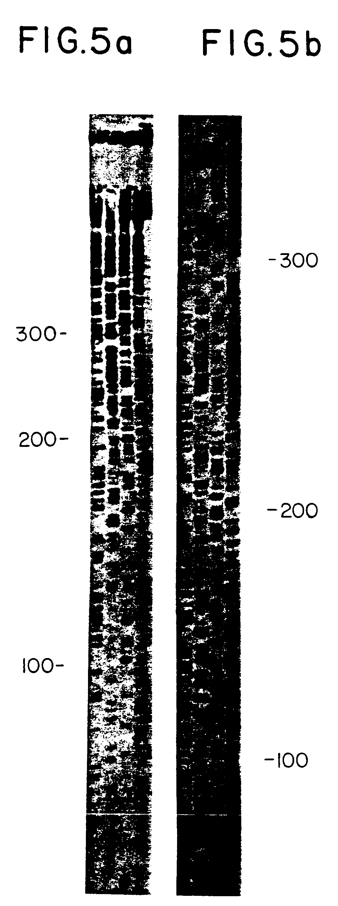

Figure 5 shows the chemiluminescent detection (using Lumi-Phos™, Lumigen, Inc., Detroit, MI) of two full size M13mp8 DNA sequence blots: (a) using standard electrophoresis/blotting, (b) using DBE. Both blots were processed as described in Examples 3 and 4, and exposed to a Fuji X-Ray film for one minute. The order of the DNA sequence from left to right is A, G, C, T. The length of the DNA sequence is indicated alongside in number of nucleotides.

## Detailed Description

This invention pertains to the use of chemiluminescence to detect nucleic acid molecules. According to the method, a nucleic acid is provided which is linked covalently or non-covalently to a compound that can generate chemiluminescence. The compound itself can emit light after inducement by an appropriate stimulus (for example, pH, temperature and chemical reactions, including enzymatic reactions). Alternatively, the compound may act on another compound to generate chemiluminescence. Preferably, the chemiluminescence generating compound is an enzyme which can induce an appropriate substrate to become chemiluminescent. Some suitable enzymes are alkaline phosphatase, acid phosphatase, peroxidase, galactosidase, glucose oxidase, luciferase, aryl esterase, sulfatase, urease, acetylcholinesterase, chymotrypsin, trypsin, aminopeptidase and lipase.

The chemiluminescence generating compound which is linked to the nucleic acid is induced to generate chemiluminescence, thereby allowing the nucleic acid to be detected. When the compound is an enzyme, it is contacted with an appropriate chemiluminogenic substrate. The chemiluminescence is indicative of the presence of the nucleic acid, and the intensity of the emitted light is indicative of the amount present. As mentioned, in a preferred embodiment, the compound that can generate chemiluminescence is an enzyme which triggers a chemiluminogenic substrate to generate chemiluminescence. The chemiluminogenic substrate is a stable but enzymatically triggerable, substituted 1,2-dioxetane. When a substituted 1,2-dioxetane is triggered by an appropriate enzyme, the enzyme cleaves the substituent on the dioxetane to form an unstable negatively charged dioxetane. The negative charge causes the dioxetane to decompose via an intramolecular electron transfer to form a biradical and light. See Schaap, A.P., European Patent Application 0254051 (1987). Several substituted 1,2-dioxetanes and their corresponding enzymes are shown in Figure 1. The preferred enzyme is alkaline phosphatase used in connection with a phosphate containing 1,2-dioxetane.

A compound that can generate chemiluminescence can be incorporated into a nucleic acid by chemically attaching it to one or more sites on the nucleic acid. For example, a chemiluminescence generating compound can be attached to the nucleic acid at the 5' and/or 3' terminus; at one or more of the nucleobases or to one or more sites on the phosphodiester backbone. The compound can be attached to the nucleic acid via organic linker molecules, biotin/avidin (streptavidin) bridges and bispecific antibodies. Methods of attaching the compound and molecules used to facilitate the attachment are described in more detail below.

The chemiluminescence can be amplified to improve sensitivity of detection. For example, the intensity of the light emitted by the chemiluminescence reaction can be amplified by incorporating a number of chemiluminescence generating molecules at multiple sites on the nucleic acid. As previously described, the chemiluminescence generating compound can be attached to the nucleic acid at the 5' terminus, the 3' terminus, at one or more sites on the nucleobases or to one or more sites on the phosphodiester backbone. Specific means by which chemiluminescence can be amplified are described in detail below.

In addition, an enhancer molecule can be used in conjunction with the chemiluminescence generating compound to further amplify the intensity of chemiluminescence. An enhancer molecule is a molecule which is excited by light emitted by the chemiluminescent compound, and emits light in its excited state. Preferably, the enhancer molecule is a fluorophore such as the class of fluoresceins, coumarins and rhodamines, and is used in combination with a carrier molecule. The carrier molecule allows close proximity of the fluorophore with the chemiluminescent generating compound. Most preferred of the fluoresceins is the parent fluorescein molecule. Two commercially available products which enhance chemiluminescence are Lumi-Phos™ (Lumigen, Inc., Detroit, MI) and Jade Green Intensifier (Quest Systems, Inc., Bedford, MA).

In nucleic acid sequencing procedures, a nucleic acid can be linked to a compound that can generate chemiluminescence in a variety of ways depending upon the nucleic acid sequencing method. A nucleic acid primer can be modified at the 5' terminus for attaching a compound that can generate chemiluminescence thereto. Likewise, a nucleic acid chain terminator, such as a dideoxynucleoside triphosphate, can be modified for attaching a chemiluminescence generating compound thereto. The compound can then be linked to the primer or terminator after the products of the sequencing reaction are separated. When the nucleic acid is a probe, for example, in hybridization reactions, the chemiluminescence generating compound can be incorporated into the nucleic acid at one or more sites on the nucleic acid. When more than one chemiluminescence generating compound is attached to the nucleic acid, the intensity of light emitted by the chemiluminescence reaction can be enhanced, as described previously.

Prior to inducing the chemiluminescent reaction, the nucleic acid linked to a chemiluminescence generating compound can be affixed to a solid support using known transfer techniques or DBE. Any porous or non-porous solid support can be used in the detection methods of this invention. However, it is advantageous to use a flat surface to facilitate covalent or non-covalent attachment of the nucleic acid to the solid surface. Several suitable solid supports are nylon membranes, nylon membrane derivatives, polyvinylidene fluoride (PVDF) membranes and derivatives thereof.

The present invention further pertains to a method of sequencing nucleic acids using chemiluminescent detection. In one embodiment, a nucleic acid primer, complementary to a region of DNA to be sequenced, is modified for attaching a compound that can generate chemiluminescence thereto. In another embodiment, the chain terminator, representing the 3' terminus of the nucleic acid, is modified for attaching a chemiluminescence generating compound thereto. Preferably, the modified primer or modified terminator has the formula, A-X-Sp-Y-$L^1$, wherein A,X,Sp,Y and $L^1$ are described in Schemes 1, 2 and 3. The primer is then hybridized to the DNA to be sequenced and extended with deoxyribonucleotides using the DNA to be sequenced as a template. The extension reactions are terminated with dideoxyribonucleoside triphosphates to produce DNA molecules whose lengths depend on the point of termination from the primer. The DNA molecules generated by each of the extension reactions are separated according to size using known separation techniques. The separated molecules are then affixed to a solid support whereby a compound that can generate chemiluminescence is attached to the separated molecules at the modified primer and/or modified terminator. The resulting DNA molecules are contacted with an appropriate stimulus, such as a chemiluminogenic substrate, to cause the compound to generate chemiluminescence. The chemiluminescence is detected to obtain a pattern of the separated DNA molecules according to size, wherein the amount of light emitted is indicative of the presence and amount of nucleic acid. The nucleotide sequence is then determined based upon the pattern of separated DNA molecules.

Alternatively, separated molecules from the sequencing reaction are linked to a bridging unit (M) (as shown in Table 1). The chemiluminescence generating compound is then linked to M directly or via a linking unit, $L^2$. The compound is caused to generate chemiluminescence and the chemiluminescence is detected as described above.

In another embodiment, DNA to be sequenced is modified at the 5' or 3' terminus for attaching a compound that can generate chemiluminescence. Preferably, the modified DNA has the formula, A-X-Sp-Y-$L^1$, wherein A, X, Sp, Y and $L^1$ are described in Schemes 1, 2 and 3. The method of modifying the 5' or 3' terminus is described in detail below. The DNA is subjected to a set of separate chemical reactions wherein the DNA is reacted with a reagent which specifically modifies one or two of the four nucleoside bases under conditions wherein only a few bases in any DNA molecule are modified. The DNA is then contacted with a reagent which cleaves the DNA at the modified base. After the cleaved molecules are separated and affixed to a solid support, a compound that can generate chemiluminescence is attached directly to the molecules at the modified 5' or 3' terminus, or is attached to the molecules via a bridging unit. A chemiluminescent reaction is induced by contacting the molecules with an appropriate stimulus to produce chemiluminescence. The chemiluminescence is detected wherein the light emitted is indicative of the

presence and amount of the nucleic acid. The nucleotide sequence is therein determined based upon the pattern of DNA molecules.

In yet another embodiment, DNA sequencing methods (e.g., Sanger chain extension method and Maxam and Gilbert degradative method) can be used in connection with multiplexing techniques to simultaneously determine DNA sequences of a number of pooled DNA molecules. Using multiplex techniques, DNA molecules produced either by chain extension or by chemically degrading the DNA to be sequenced, are separated according to size and affixed onto a solid support. The separated molecules are then probed with DNA probes specific for individual DNA molecules to be sequenced wherein the probe has a nucleic acid sequence complementary to the DNA and is covalently linked to a compound that can generate chemiluminescence at one or more sites located thereon. The probe is hybridized to the separated DNA molecules and contacted with an appropriate stimulus to cause the compound to generate chemiluminescence. The chemiluminescence is detected wherein the light emitted is indicative of the presence and amount of a specific DNA. The probing step is repeated using probes unique to each DNA to be sequenced to thereby produce a nucleotide sequence for each individual DNA molecule.

Alternatively, the probe can be modified at one or more sites located on the probe for non-covalently attaching a chemiluminescence generating compound. The modified probe is then hybridized to separated molecules which were generated in a sequencing reaction. A compound that can generate chemiluminescence is then attached to the modified probe. Methods of attaching the chemiluminescence generating compound are described in detail below.

Chemiluminescence can also be used in hybridization reactions. Specifically, a nucleic acid probe is attached covalently or non-covalently to a chemiluminescence generating compound as described above. DNA/RNA to be detected is affixed to a solid-phase and is then contacted with the probe using standard hybridization techniques. The hydridized probe is contacted with an appropriate stimulus under conditions suitable to generate a chemiluminescent reaction. The light emitted by the chemiluminescent reaction indicates the presence and quantity of the hybrid.

Nucleic acid can be sequenced using either the chemical degradation method of Maxam and Gilbert or the enzymatic/primer extension method of Sanger. Multiplexing techniques can be employed based upon either of the two sequencing chemistries. Nucleic acids are linked covalently or non-covalently to a compound that can generate chemiluminescence or that can cause another compound to generate chemiluminescence.

Covalent and non-covalent methods for attaching a compound which can generate chemiluminescence to a nucleic acid are shown in Schemes 1, 2 and 3.

Scheme 1: Covalent attachment of a compound that can generate chemiluminescence to a nucleic acid

A-X-Sp-Y-E

A is a nucleic acid;
X and Y are the same or different chemical functional groups that link the nucleic acid to E;
Sp is a chemical spacer group that provides an appropriate linking distance between A and E; and
E is a compound that can generate chemiluminescence or cause another compound to generate chemiluminescence.

Scheme 2: Non-covalent attachment of a compound that can generate chemiluminescence to a nucleic acid

$A\text{-}X\text{-}Sp\text{-}Y\text{-}L^1 + M + L^2\text{-}E \rightarrow$
$A\text{-}X\text{-}Sp\text{-}Y\text{-}L^1{\sim}M{\sim}L^2\text{-}E$

A, X, Y, Sp and E are defined above;
$L^1$ and $L^2$ are the same or different and are linking units;
~ represents a non-covalent attachment; and
M is a bridging unit.

Scheme 3: Non-covalent attachment of a compound that can generate chemiluminescence to a nucleic acid

$A\text{-}X\text{-}Sp\text{-}Y\text{-}L^1{\sim}M{\sim}E$
$A\text{-}X\text{-}Sp\text{-}Y\text{-}L^1{\sim}M\text{-}E$

A, X, Sp, Y, $L^1$, M and E are defined above.

Scheme 1 shows a nucleic acid which is covalently attached to a compound that can generate chemiluminescence. A nucleic acid used as a probe suitable for hybridization reactions can be covalently linked to a compound capable of generating chemiluminescence (E) via a spacer (Sp) and chemical functional groups (X) and (Y). X and Y can be the same or different and are chemical functional groups, such as amino, hydroxy, carboxy, mercapto, methylene, carbonyl and amido. Preferably, E is an enzyme which can be contacted with a substrate to produce a chemiluminescent reaction.

Alternatively, schemes 2 and 3 show the non-covalent attachment of a compound that can generate chemiluminescence to a nucleic acid. The symbol, ~, is herein used to represent non-covalent attachment. The symbol, -, is used to represent covalent attachment. The nucleic acid is linked to a spacer (Sp) via a chemical functional group (X). The spacer is further connected to a linking unit ($L^1$) via a chemical functional group (Y) which can be the same or different as X. Linking unit ($L^1$) can be selected from a variety of molecules including biotin, immunobiotin, specific lectin-binding sugars, lectins, small molecules including steroids, peptides, a 2,4-dinitrophenylated amino functional group, haptens, hapten or biotin specific antibodies. When $L^1$ is a hapten or biotin specific antibody, it is linked to the nucleic acid after the products of the sequencing reaction are separated using known separation techniques. In a preferred embodiment, A is DNA, $L^1$ is biotin, M is avidin/streptavidin, $L^2$ is biotin, E is alkaline phosphatase and X-Sp-Y is C(O)NH-$(CH_2)_6$NH.

If the chain extension method of Sanger is used, the nucleic acid primer and/or nucleic acid terminator, such as dideoxynucleoside triphosphate, is covalently linked to a linking unit ($L^1$) via X-Sp-Y. After the products of the sequencing reaction are separated using known separation techniques, a compound capable of generating chemiluminescence (E) is linked to the separated reaction products via $L^1$.

As shown in Scheme 2, linking units $L^1$ and $L^2$ can be non-covalently linked to E via a bridging unit (M). Alternatively, the bridging unit M can be a compound which can link the nucleic acid indirectly to E without $L^2$ (Scheme 3). Several suitable bridging units are biotin, avidin/streptavidin, biotin specific antibody, hapten, hapten specific antibody, lectin, lectin specific sugar and bispecific antibodies.

The bridging unit can be further linked to other molecules which can itself be linked to an additional compound that can generate chemiluminscence (E). By adding additional bridging units, the intensity of light emitted from a chemiluminescent reaction can be amplified. Amplification results since each chemiluminescence generating compound adds to the intensity of light generated in the reaction. Table 1 shows some combinations of linking and bridging units for the non-covalent attachment of E. It is not meant to be comprehensive.

A nucleic acid can function as a primer using the chain extension method of Sanger or as a probe to detect a pooled sequencing pattern using the multiplex DNA sequencing approach of Church using either the Sanger or Maxam and Gilbert method to perform the sequencing reactions. Scheme 4 shows three ways in which the chemiluminescence generating compound (E) can be attached to the nucleic acid when it is functioning as a primer/probe (A1, A2 and A3).

TABLE 1

Non-Covalent Binding of E

| | a) | b) | c) | d) | e) | f) | g) | h) | i) | j) |
|---|---|---|---|---|---|---|---|---|---|---|
| $L^1$ | Biotin | Biotin | Biotin Specific Antibody | Biotin | Biotin | Hapten Specific Antibody | Hapten | Lectin | Lectin Specific Sugar | Enzyme Specific Antibody |
| $L^2$ | E | — | — | Biotin | Anti-Antibody | — | — | Lectin | — | — |
| M | Bispecific Antibody | Biotin Specific Antibody | Biotin | Avidin/ Strept-avidin | Biotin Specific Antibody | Hapten | Hapten Specific Antibody | Lectin Specific Sugar | Lectin | — |

Scheme 4: Modification of Oligonucleotide

A1:

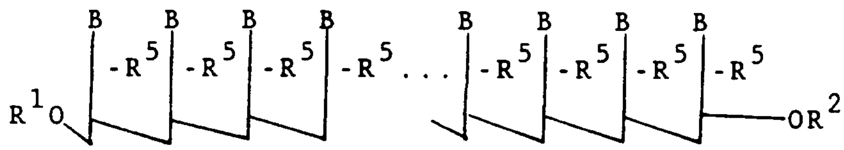

$R^1$ and $R^2$ are H,

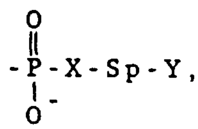, 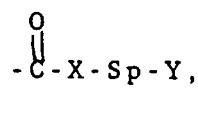, or 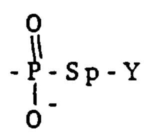

X is O, S, NH, $CH_2$, NHNH, CO, NHCO, or SS;
$R^5$ is H or OH;
Y is OH, $NH_2$, SH, CO, $CO_2Z$, $CO_2H$, Br, Cl, F, HgCl, azide, CO- imidazole, NH-$NH_2$, CO-$NHNH_2$, or N-maleimido;
Sp is $(CH_2)_n$, $(CH_2)_n(C_6H_4)_m$, $[NH(CH_2)_nCHR^6]_m$,

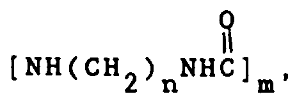,

$NH(CH_2)_n$, $[NH(CH_2)_nOC]_m$,

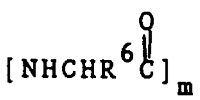, 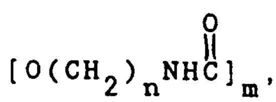, 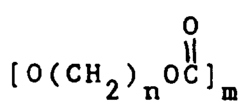,

$[R^6CH(CH_2)_nNH]_m$,

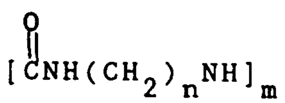,

$(CH_2)_nNH$,

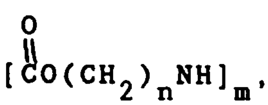, 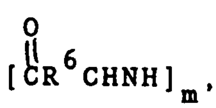, 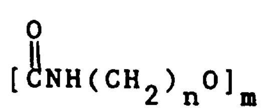,

or 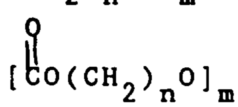

wherein $R^6$ is H, $(CH_2)_nNH_2$, $(CH_2)_nCOOH$, or $(CH_2)_nSH$;
Z is an alkyl, aryl, aralkyl group forming preferentially active ester bonds, including 4-nitrophenyl, pentafluorophenyl, 3,5-dichlorophenyl, methoxymethyl, hydroxybenztriezolyl and N-hydroxysuccinimidoyl;
n is an integer from 1 to 20 and m is an integer from 1 to 6; and

B is a nucleobase selected from the group consisting of adenine, guanine, thymidine, cytosine and uracil.

A2:

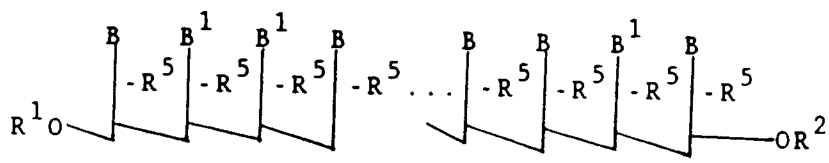

$R^1$, $R^2$, $R^5$ and B are defined above.

$B^1$ is 5-modified pyrimidine, 8-modified purine base, 7-modified N7-deaza adenine, 7-modified N7-deaza adenine;

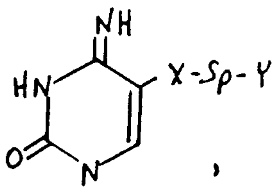 , 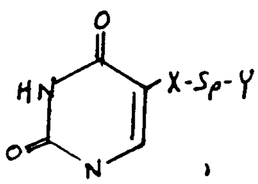 , 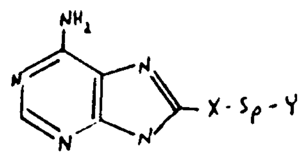 , 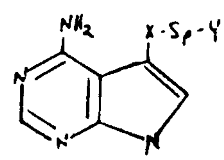 ,

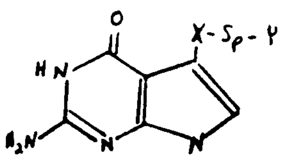 or 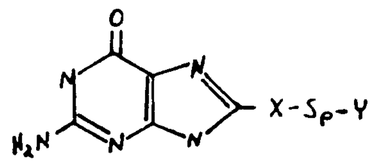

X, Sp and Y are defined above.

A3:

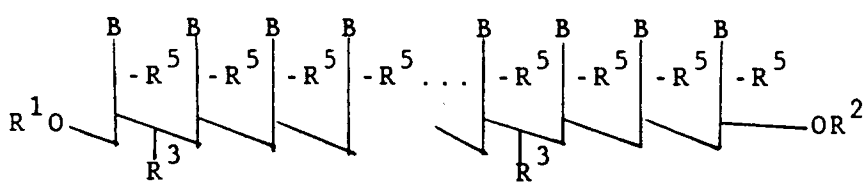

$R^1$ is H or

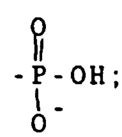

$R^2$ is H;

$R^3$ is

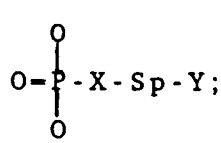

$R^5$ is H or OH; and

X, Y, Sp and B are defined above.

In the Sanger sequencing process short nucleic acids are used as primers which are enzymatically extended by various enzymes including E. coli DNA polymerase I, the large fragment of E. coli DNA polymerase I (Klenow fragment), T4 and T7 polymerases, sequenase, thermostable DNA polymerases including Taq polymerase and a mixture of the four deoxynucleoside triphosphates and in four different chain extension reactions one of the respective dideoxynucleoside triphosphates as base specific chain terminators. A compound which can generate chemiluminescence, for example, an enzyme, can be coupled to the nucleic acid by the covalent attachment method or the non-covalent attachment method via the nucleobases (A2 in scheme 4 and A4 in scheme 5), the modified phosphodiester backbone (A3 in scheme 4) or the 5'- or 3'- termini (A1 in scheme 4). Scheme 5 shows various ways in which a deoxyribotriphosphate can be linked to a compound which can generate chemiluminescence.

Scheme 5: Modification of (deoxy)ribotriphosphate

A4:

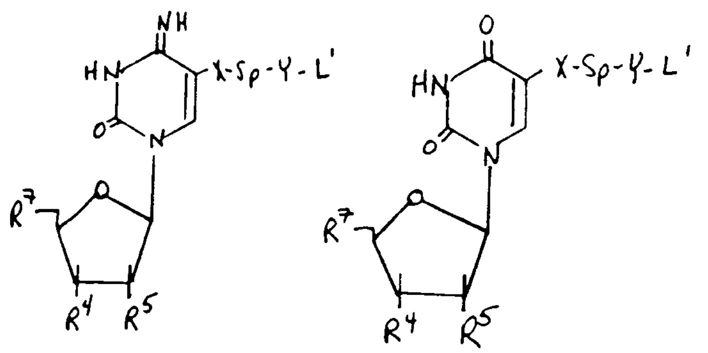

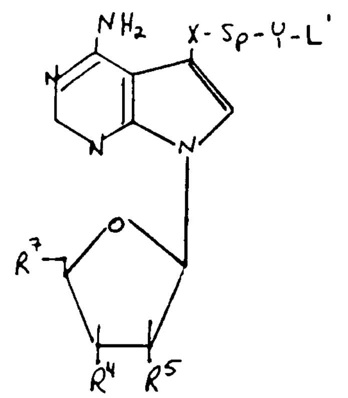

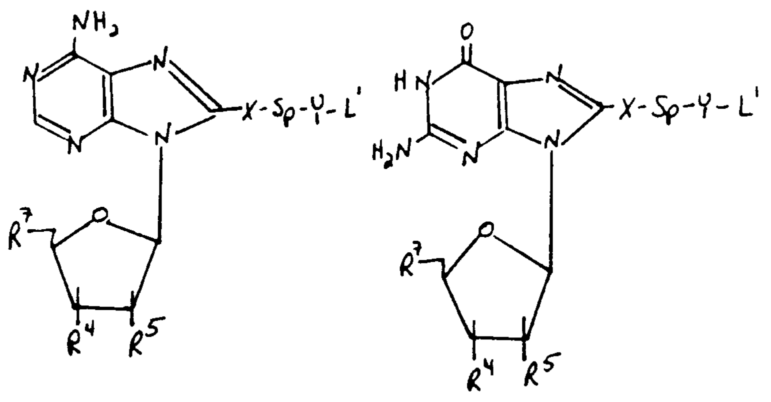

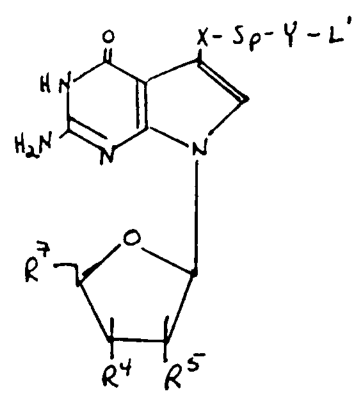

A5:

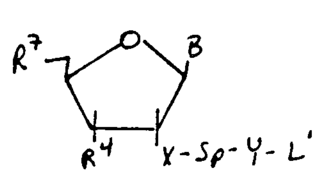

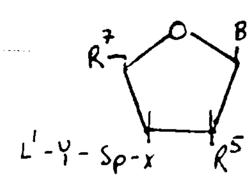

B is a nucleobase selected from the group consisting of adenine, guanine, thymidine, cytosine, uracil, 7-deaza adenine, and 7-deaza guanine;

$R^4$ and $R^5$ are H or OH;

$R^7$ is

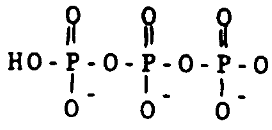, 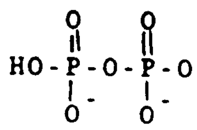, or 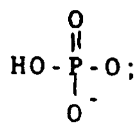;

and

Sp, X, Y and $L^1$ are defined above.

X and Y can be selected from a number of functional groups including amino, hydroxy, phosphate mercapto, carboxy, ester (preferentially active ester), chlorine, bromine, iodine, HgCl, hydrazino, azido, acyl hydrazido, acyl imidazole, N-(carbonyloxy) imido, amido, methylene, carbonyl, N-maleimido and iminoester groups. Phosphate groups can be incorporated into the nucleic acid by known enzymatic reactions or by chemical oligodeoxynucleotide synthesis. The carboxy groups are linked to the nucleic acid by chemical reactions after chemical oligonucleotide synthesis.

The spacer (Sp) can be linked to a nucleic acid through a chemical functional group (X). The spacer (Sp) is an organic molecule which functions to introduce the appropriate distance between the nucleic acid and E to prevent steric hindrance and rotational problems. Sp can be selected from the group consisting of an alkyl, alkenyl, and alkynyl having one to twenty carbon atoms, an aralkyl, aralkenyl, and aralkynyl having one to twenty carbon atoms in the alkyl/alkenyl/alkynyl part and up to six carbon atoms in the aryl unit. Methyl, ethyl or isopropyl groups can be optionally added to reduce the free rotational mobility in the alkyl/alkenyl/alkynyl chain. The aryl groups may optionally be substituted by chlorine, fluorine, bromine, cyano, nitro and lower alkyl groups, such as methyl, ethyl, isopropyl. Sp can also be an amino acid or polyamino acid, an alkyl urethane or polyalkylurethane, an alkylurea or polyalkylurea or alkylcarbonate or polyalkylcarbonate, in which the polymer is 1-20 units. It is also possible to introduce the moiety X-Sp-Y by reacting the nucleic acid with a homobifunctional (X = Y) or heterobifunctional reagent (X≠Y) according to known reactions.

When the nucleic acid termini are the attachment points, only the 5'-terminus can be modified (by attaching the X-Sp-Y moiety to the terminus) if it is used as a Sanger sequencing primer. The chemiluminescence generating compound is then linked to the moiety after the products of the sequencing reaction have been separated. For multiplex DNA sequencing, however, in which the nucleic acid functions as a probe, the chemiluminescence generating compound can be covalently or non-covalently linked at one or more sites selected from the 5' terminus, 3' terminus, one or more of the nucleobases, and one or more sites on the phosphodiester backbone. As a result of attaching the chemiluminescent generating compound to multiple sites on the probe, the intensity of chemiluminescence can be increased.

Modified internucleotide bonds (A3 in scheme 4) can also be used to establish attachment points for E. These modified internucleotide bonds are introduced using standard chemical techniques. (Sinha, N.D. et al., Nucleic Acids Res. 12:4539 (1984)). The use of suitably protected nucleoside H-phosphonates (Froehler, B.C. and M.D. Matteucci, Tet. Lett. 27:469-472 (1986); Froehler, B.C. et al., Nucl. Acids Res. 14:5399-5407 (1986); Garegg, P.J. et al., Tet. Lett. 27:4051-4054 (1986); Garegg, P.J. et al., Tet. Lett. 27:4055-4058 (1986)) or 1,1-dimethyl-2-cyanoethyl nucleoside phosphoamidites (Nielsen, J. and M.H. Caruthers, J. Am. Chem. Soc. 10:6275-6276 (1988)) during standard chemical oligodeoxynucleotide synthesis allows various Sp-Y moieties via P-C, P-O, P-S and P-NH or P-N bonds to be introduced. The nucleic acids may be partially or completely modified at their internucleotide bonds.

In one embodiment, the nucleic acid primer/probe has an attachment site at the heterocyclic nucleobase (A2 in scheme 4, A4 in scheme 5). This configuration, however, should not interfere with the necessary property of the primer/probe to specifically hybridize via Watson-Crick base pairing with the complementary target nucleic acid. For the sake of clarification, it should be noted that the heterocyclic bases are linked to the ribose/deoxyribose moiety via the anomeric C1' carbon atom; the pyrimidine heterocycle is attached to the sugar via the N1 and the purine heterocycle via the N9 atom. In case of the pyrimidine moiety, the X-Sp-Y-L unit can only be linked to the C5 carbon atom; in case of the purine ring it has to be attached at the C8 carbon atom, although this is sterically not optimal. Another way to link the X-Sp-Y-$L^1$ unit to the purine ring system is to use N7-deaza adenine and N7-deaza guanine nucleosides in which case the X-Sp-Y-$L^1$ unit can be attached to the C7 carbon atom. For the synthesis of the described base-modified primer/probe oligonucleotides suitable protected synthons have to be used (A6 in scheme 6). Depending on the chemical synthetic strategy chosen, a compound that can generate chemiluminescence should be linked to the nucleic acid after completion of the oligonucleotide synthesis. Thus an intermediate configuration X-Sp-Y' compatible with the synthetic strategy has to be selected. The exocyclic amino

groups and the 5' OH group have to be protected using known amino and hydroxyl protecting groups, such as acyl and amidine groups to protect the exocyclic amino group and various trityl groups to protect the 5'OH group. The term protecting group is intended to mean a structure that can bind to the amino or the 5' OH group and, thus, render unreactive, the group which it binds to. In the case of oligoribonucleotide synthesis the 2'OH groups have to be protected using known protecting groups such as tetrahydrofuranyl, tetrahydropyranyl, o-nitrobenzyl and various silyl protecting groups. To provide for an efficient coupling function either phosphoamidites or H-phosphonates can be used. Scheme 7 shows examples of various ways nucleic acids can be modified for attachment of a compound which can generate chemiluminescence.

Scheme 6: Synthons for base-modified oligonucleotides

A6:

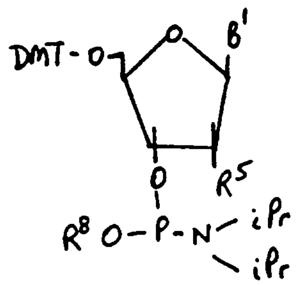

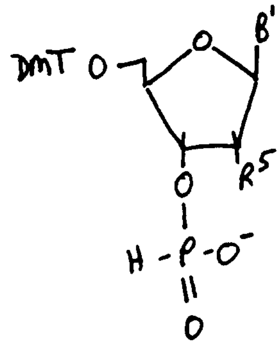

$B^1$

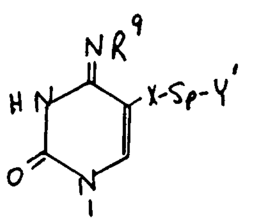

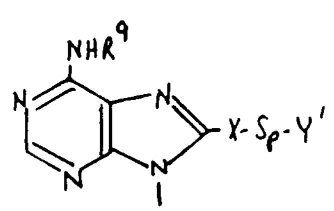

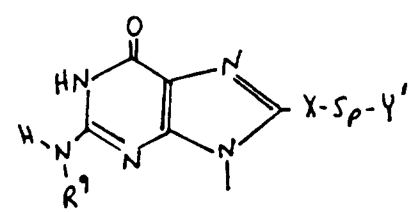

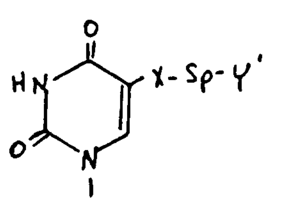

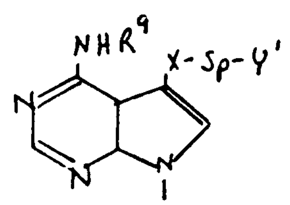

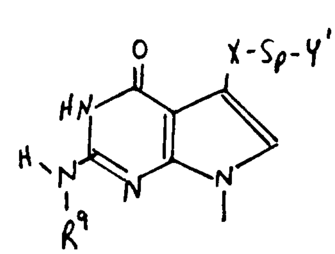

$B^1$, X and Sp are defined above;
$R^5$ is H or $OR^{10}$;
$R^8$ is $CH_3$, $CH_2CH_2CN$, $C(CH_3)_2CH_2CN$;
DMT is 4,4-dimethoxytrityl;
$R^9$ is an amino-protecting group;
$Y^1$ is a Y group as defined above which is linked to a protecting group suitable for oligonucleotide synthesis; and
$R^{10}$ is a H or OH protected with a group suitable for oligonucleotide synthesis.

Scheme 7: Modification of nucleic acids for attachment of a compound that can generate chemiluminescence

a) 5'-end attachment

$L^1$ or

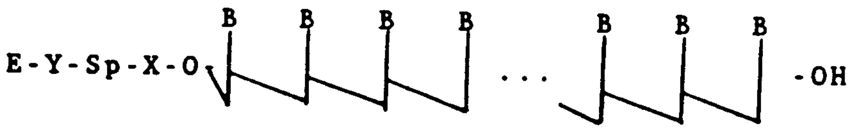

b) internal attachment

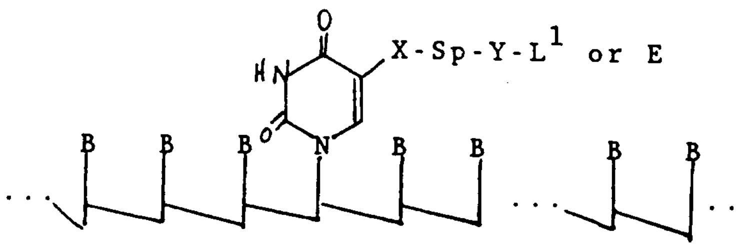

X, Y and Sp are defined above; and

$L^1$ or E is selected from the group consisting of enzymes, biotin, antibodies and haptens, wherein $L^1$ or E may be linked to the nucleic acid through a spacer unit as previously defined.

Any combination of attachment of the X-Sp-Y-$L^1$ or E moiety at the 5' or 3' termini, heterocyclic base and internucleotide bond is possible and is embraced by the invention. Attachment of the moiety at various locations on the nucleotide allows the signal to be amplified due to the presence of multiple attachment sites. As a result, detection sensitivity is increased. In particular circumstances, it is advantageous to use oligoribonucleotides rather than oligodeoxyribonucleotides. The above-described attachment methods can be modified depending upon the presence of protected or unprotected 2'-OH functionalies. It should be understood, however, that the point of attachment and molecules used to attach the chemiluminescence generating compound depend upon the sequencing and separation methods used. Additionally, protein molecules, including antibodies and enzymes, must be attached after the products of the sequencing reaction have been separated.

Points for modifying and attaching a compound that can generate chemiluminescence are summarized as follows:

Sanger chain extension method: A nucleic acid primer can be modified for attaching a chemiluminescence generating compound at one or more sites located thereon, selected from the 5' terminus, one or more of the nucleobases and one or more sites on the phosphodiester backbone. Alternatively, the chain terminator can be modified at the nucleobase, the sugar or combination thereof.

Maxam and Gilbert chemical degradation: A nucleic acid to be sequenced can be modified at either the 5' terminus or the 3' terminus, selected from the nucleobase and the sugar or combination of nucleobase and sugar.

Hybridization probes: When the nucleic acid is a hybridization probe, the probe can be linked to a compound that can generate chemiluminescence at one or more sites located thereon, selected from the 5' terminus, 3' terminus, one or more of the nucleobases and one or more sites on the phosphodiester backbone.

Another method for incorporating a chemiluminescence generating compound into the DNA sequencing process is by modifying nucleoside triphosphates (A4 and A5 in scheme 5). In this case, base-modified deoxynucleoside triphosphates or ribonucleoside di- or triphosphates (depending on the enzyme applied) can be enzymatically incorporated into DNA or RNA respectively using known enzymes and procedures. The attachment site for the heterocyclic bases is determined using the same considerations as discussed above. When A4 is a chain terminator using the Sanger DNA sequencing method then $R^4$ is H and $R^5$ is H or OH. As previously discussed, modifying the chain terminator for subsequent attachment of a chemiluminescence generating compound advantageously reduces the background level in DNA sequencing. If nucleoside triphosphates are used as terminators, the attachment site of X-Sp-Y-$L^1$ can also be positioned within the sugar moiety either at the 3' or 2' position (A5 in scheme 5). Here the selection of the spacer is governed by the substrate properties of A5 for DNA/RNA polymerases. In the case of chain terminators, all combinations of base and sugar modifications can be selected in order to increase the signal by multiplying the attachment sites. All such combinations are included within the scope of the invention.

The compound which can generate chemiluminescence can be an enzyme, such as alkaline phosphatase, acid phosphatase, peroxidase, galactosidase, glucose oxidase, luciferase, aryl esterase, sulfatase, urease, acetylcholinesterase, chymotrypsin, trypsin, aminopeptidase and lipase. When the nucleic acid is covalently or non-covalently attached to an enzyme, it is contacted with the chemiluminogenic substrate to produce a chemiluminescent reaction.

Chemiluminescence can be enhanced by attaching additional E groups to the nucleic acids to amplify light emitted from the chemiluminescent reaction. This mode of enhancement is herein defined as direct chemiluminescent enhancement since the light which is detected results from the chemiluminescent reaction itself. This can be achieved either by using polymerized complexes consisting of multiple E groups instead of one individual molecule or by using an antibody enhancement mode as employed in immunological and clinical procedures such as ELISA (enzyme-linked immunosorbent assay). In the latter case, a specific antibody 1 (Ab 1) against linking unit $L^1$ or compound that can generate chemiluminescence (for example, an enzyme or enzyme complexes) is bound to $L^1$ or E. To Ab 1 multiple second antibodies (alpha Ab 1) can be bound which was previously conjugated to E using known immunological techniques. Direct chemiluminescence is enhanced by the specific attachment of more compounds which can generate chemiluminescence (for example, an enzyme) per DNA target molecule.

In addition to direct chemiluminescence, chemiluminescence can be significantly amplified using enhancer molecules which can themselves emit light via intermolecular energy transfer. The use of such enhancers produces an indirect chemiluminescence reaction since the light which is detected is from the enhancer molecule. Suitable enhancer molecules are described above. Enhancers, suitable antibodies and several substituted 1,2-dioxetanes are commercially available (Lumigen, Inc. Detroit, MI; Quest Systems Inc., Bedford, MA, Amersham International, UK). Preferably, chemiluminescence can be amplified using both direct chemiluminescence and indirect chemiluminescence enhancement.

In the preferred embodiment, the chemiluminescent detection of DNA is carried out using an enzyme catalyzed reaction with a stable but enzymatically triggerable 1,2-dioxetane (see Figure 2). The target DNA is immobilized on a nylon membrane by employing various known techniques such as UV crosslinking and covalent and non-covalent chemical attachment. Though nylon membranes are shown in the example, any porous or non-porous solid support can be used in this invention. Preferably, the solid support has a flat surface to facilitate the attachment of a nucleic acid. As shown in Figure 2, the oligodeoxynucleotide primer A (scheme 1 and 2) is modified at its 5' end with biotin ($L^1$) via 1-6-diaminohexane as a spacer Sp. Alkaline phosphatase (E) is also coupled to biotin as a linking unit ($L^2$) and attached via streptavidin as a bridging unit M to the oligonucleotide primer. When the nucleic acid, which is linked to a compound that can generate chemiluminescence, is contacted with the chemiluminogenic substrate, the phosphate moiety is cleaved off by the action of the enzyme to generate chemiluminescence.

In order to test the sensitivity and specificity of the system, dot blots of 1:5 serial dilutions of single stranded M13mp8 DNA were prepared and hybridized with the oligodeoxynucleotide Bio15M13 (see Example 2). The results after chemiluminescent detection (see Example 3) are shown in Figure 3. After the first detection cycle (Figure 3, 1a - d) the probe was stripped from the membrane (Figure 3, a) and the membrane was rehybridized (Figure 3, 2a - d). Under these conditions as little as 0.4 fmoles DNA per dot (5 mm in diameter) could be detected in less than 1 minute exposure (Figure 3, 1c - d). The first two dots, containing 50 and 10 fmoles target DNA respectively, could easily be detected by eye in the darkroom. The probe or the signal can quantatively be stripped for the membranes as shown in Figure 3, e. Even five minutes exposure time showed no remaining signal. In order to determine the maximum possible number of probing cycles per membrane, the same membrane strip was subjected to a total of 40 hybridization/stripping cycles. Figure 3 (20 and 40) show the result after re-probing cycle 20 and 40, respectively. The membrane was exposed to the same period of time after probing number 1 and 2. This experiment simulates the multiplex DNA sequencing technique.

In another experiment, a DNA sequence blot was prepared using Bio15M13 oligonucleotide in primer extension sequencing reactions (see Example 4). The reaction products were separated and blotted using direct blotting electrophoresis (DBE) (see Example 4). After processing the blot as described in Example 3, the DNA sequence pattern was detected in a luminometer using a Polaroid 57 film to capture the image. Figure 4 shows the result of a chemiluminescent detected DNA sequence band pattern at various exposure

times ranging from 1 sec (Figure 4, a) to 45 sec (Figure 4, d). After 1 sec exposure time the entire band pattern is clearly visible with virtually no background interference.

To demonstrate that chemiluminescent detection as presented in this invention is superior in sensitivity and detection speed over all existing methods, two full size DNA sequence blots were prepared and processed, one using the standard electrophoresis/blotting technique (Figure 5a) and one using the DBE technique (Figure 5b). The results presented in Figure 5 show the M13mp8 sequence ranging from 50-350 nucleotides. In both cases, the DNA was blotted onto a nylon membrane (BIODYNE™, Pall, NY) and was exposed for one minute to a Fuji X-Ray film. The exposure was done by placing the x-ray film directly onto the plastic bag in which the blot was processed. Within the variation due to the sequencing chemistry (Klenow enzyme), the intensity of the individual bands is uniform over the entire molecular weight range of standard dideoxy sequencing reaction products.

The chemiluminescence detection chemistry as described in this invention for DNA hybridization using either multiplex DNA sequencing or standard dideoxynucleoside DNA sequencing is sensitive and has several advantages over existing radio-isotopic, fluorescent and colorimetric methods:

1) Stability: Biotin-linked oligonucleotides and sequencing reactions are not subject to decay, radiolysis or light bleaching. They are stable indefinitely if kept under proper conditions. This makes the chemiluminescent and any biotin based chemistry especially suitable for an economic way of batch processing large number of samples, e.g. for multiplex DNA sequencing.

2) Detection time: The high yield of the phosphatase catalyzed light reaction allows for very short detection times, usually in the range of seconds for an entire sequence blot. This is a vast improvement when compared to the range of hours needed for the detection using radio-isotopes or colorimetric staining. In addition, it is possible to read the DNA sequence directly off the membrane with a cooled CCD camera thereby further increasing the speed with which the data can be collected. Unlike the present invention, the use of on-line fluorescent detection for sequencing is disadvantagous due to the length of time it takes to generate the entire sequence. Therefore, the use of fluorescent detection for large scale DNA sequencing, i.e. multiplex DNA sequencing would not be feasible.

3) Materials: The methods of the invention are fast and highly sensitive and provide a convenient way to sequence DNA. Most of the material and equipment necessary for carrying out the method are available in any biochemical/molecular biology laboratory.

Furthermore, the process of the invention is more economical than fluorescent sequencing since many membranes can be processed simultaneously, thus eliminating the need for very special, expensive and complex equipment (laser, optics, electronics) as is used in fluorescent sequencing. A further advantage of this process is the compatability with the widely used method of radioactive detection for DNA sequencing. In both cases the permanent image of the sequence data is an x-ray film which can be read either manually (e.g. with the help of a sonic digitizer) or with a commercially available automatic film reader.

Example 1: Biotinylation of the Oligonucleotide

The synthesis of 5'-T-C-C-C-A-G-T-C-A-C-G-A-C-G-T-3' was conducted on a 1.0 μmole scale using β-cyanoethyl phosphoramidites (Sinha, N.D. et al., Nucleic Acids Res. 12:4539 (1984)) and a MilliGen 7500 DNA synthesizer. The support-bound detritylated oligomer was reacted with 1,1'-carbonyldiimidazole and 1,6-hexanediamine as described by Wachter et al., (Wachter et al., Nucleic Acids Res. 14:7985 (1986)) to give after HPLC purification 108 $A_{260}$ units of $H_2N(CH_2)_6NHC(O)$-(T-C-C-C-A-G-T-C-A-C-G-A-C-G-T). Reaction of the amino-terminated oligonucleotide (25 $A_{260}$ units) with the N-hydroxysuccinimide ester of d-biotin was carried out as described by Coull et al.. (Coull, J. et al., Tet. Lett. 27:3991 (1986)). The biotinylated oligomer was isolated from the reaction mixture by reversed-phase chromatography. Fractions containing the product (referred to as Bio15M13) were combined to yield 13.8 $A_{260}$ units of the purified oligonucleotide. For hybridization experiments the oligonucleotide can also be enzymatically labeled at the 3'-end with the terminal deoxynucleotidyl transferase and Bio11-dCTC (Kumar, A. et al., Anal. Biochem. 169:376 (1988)).

Example 2: Dot-blot hybridization

Dilutions of M13mp8 DNA (50,10,2,0.4,0.08 and 0 fmoles per 100 μl) were made in TE buffer (10 mM Tris-HCl, pH8,1 mM $Na_2EDTA$). Nylon membranes (e.g. Nytran™) were pre-wet in $H_2O$ and PVDF membranes (polyvinylidene difluoride, e.g. Immobilon-N). The PVDF membranes were pre-wet first in 25% ethanol and then in $H_2O$ before being placed into a homemade vacuum filtration device (well size, 4 mm in diameter). Each well was washed with 100 μl TE buffer under moderate vacuum before the DNA dilutions

were applied. After a 100 µl TE wash the DNA was UV-crosslinked (for 1 min with 2100 µwatt per $cm^2$ membrane) to nylon membranes or baked for 10 min at 80°C for PVDF followed by a pre-wetting procedure as described above. The hybridization was essentially performed as described by Church and Kieffer-Higgins (Science 240:185 (1988)) and several membranes were processed in parallel. Prehybridization was performed in a heat sealed plastic bag with 100 µl PBS, (7.3 g/l NaCl, 2.36 g/l $Na_2HPO_4$, 1.3 g/l $NaH_2PO_4.2H_2O$), containing 5% SDS (prehybridization solution) per $cm^2$ of membrane for 30 min at 42°C. The prehybridization solution was replaced, 200 f moles of probe (Bio15M13) were added and the reaction was allowed to hybridize over night at 42°C. Unhybridized probe was removed by washing the membrane 8 times (15 min each, at room temperature) in an excess volume of PBS, 0.5% SDS in a plastic tray with moderate shaking. If the chemiluminescent detection (see Example 3) was not carried out on the same day the membranes were stored in PBS, 0.2% SDS in a heat sealed plastic bag. After the detection was complete (as described under Example 3) the probe was removed by adding 1 ml of strip solution (0.1% SDS, 2mM $Na_2EDTA$, adjusted to pH 8 with Tris base) per $cm^2$ of membrane and heating for 20 min at 80°C in a heat sealed plastic bag. This was followed by a 15 min wash in PBS, 0.5% SDS. The membranes were now ready for a second hybridization cycle or were stored in PBS, 0.2% SDS.

Example 3: Chemiluminescent detection

The DNA sequencing blots and dot-blots were processed similarly except the blocking step was omitted for the dot-blots because these membranes had been blocked during the prehybridization. All membrane manipulations were carried out wearing gloves and all incubation steps were done at room temperature. The blots were blocked with 100 µl PBS, 5% SDS per $cm^2$ of membrane for 30 min in a plastic bag. The blocking solution was drained and 100 µl per $cm^2$ of membrane of PBS-HT (PBS with 15,000 units/l heparin and 0.5% Tween 20) containing 1.0 µg/ml streptavidin were added and allowed to incubate for 10 min. After three 15 min washes in an excess volume of PBS-HT, 100 µl per $cm^2$ of membrane, PBS-HT containing 0.5 µg/ml biotinylated alkaline phosphatase was added and incubated for another 10 min. Unbound phosphatase was removed by two 15 min washes in an excess volume of PBS-HT, 0.1% (bovine serum albumin (BSA) followed by two 15 min washes in 100 mM Tris-HCl pH 9.5 100 mM NaCl, 50 mM $MgCl_2$. The membranes were washed for 5 min in carbonate buffer (50 mM $NaCO_3$, pH 9.5, 1mM $MgCl_2$). If the chemiluminogenic substrate from Quest Systems, Inc. was used the membranes were incubated for 5 min in 100 µl per $cm^2$ membrane of Jade Green Intensifier (Quest Systems, Inc., Bedford, MA). The chemiluminescent reaction was initiated by incubating the membranes in AMPPD (0.3mM to 1.6 mM 3-(2'spiroadamantane)-4-methoxy-4- (3'-phosphoryloxy)phenyl-1,2-dioxetane disodium salt) (Quest Systems, Inc.) in Jade Green Intensifier solution. If the chemiluminescent substrate from Lumigen, Inc. was used, the membranes were incubated for 5 minutes in 100 µl per $cm^2$ membrane in Lumi-Phos™ (Lumigen, Inc., Detroit, MI) (140 mg/l in 750 mM 2-amino-2-methyl-1-propanol pH 9.6, 0.8 mM $Mg(OAc)_2$, cetyltrimethylammonium bromide (CTAB) and fluorescein surfactant). After 5 min, the chemiluminescent substrate was drained and the damp membranes were sealed in the plastic bag and exposed for 1-60 sec to a Polaroid 57 film (using a luminometer, e.g. from Hoefer Scientific Instruments, San Fransisco) or x-ray film (Fuji or Kodak). The chemiluminescent reaction could be detected for up to 12 hours. The chemiluminescent substrate solution was recovered, stored at 4°C, and reused over a period of 3 weeks.

Example 4: DNA sequencing reactions

The DNA sequencing reactions were performed in microtiter plates (Falcon, #3911) as described by Bankier, A.T. et al., Methods in Enzymology 155:51 (1987)) with the following modifications. For each sequencing reaction (T,C,G & A) 2 µl single stranded M13mp8 template DNA (200 fmoles) and 2 µl primer-mix, (1.5 µl dNTP/ddNTP-mix, 0.25 µl 100 mM Tris-HCl pH 8, 50 mM $MgCl_2$, 0.25 µl Bio15M13 (500 fmoles)) were mixed (Connell C. et al., BioTechniques 5:342 (1987)). The microtiter plate was covered with saran wrap and the biotinylated primer was allowed to anneal for 30 min at 55°C. For the primer extension reaction, 2 µl enzyme mix (0.5 U Klenow in 12.5 mM dithiothreitol) were added to each reaction well. Again the microtiter plate was covered with saran wrap and incubated for 30 min at 37°C. The reactions were stopped by adding 2 µl of formamide dye (deionized formamide, 20 mM $Na_2EDTA$, 0.03% bromphenol blue, 0.03% xylene cyanol) to each well. If the reactions were analyzed the same day they were denatured for 20 min at 80°C before being loaded onto the sequencing gel. Otherwise the reactions were covered with saran wrap and stored at -20°C.

Gel electrophoresis and blotting:

A) Direct Blotting Electrophoresis (DBE). DBE was used to simultaneously perform electrophoretic separation and electroblotting onto an immobilizing matrix. A 6% polyacrylamide gel (in TBE, pH 8.8, 7.6 M urea, 17.5 cm length, 23 cm width, 0.34 cm thickness) was used as a sequencing gel with a blotting speed of 10 cm per hour. DBE was performed at a constant power of 20 watts. Following the separation the DNA was crosslinked to the wet membrane for 1 min with 2100 µwatts per $cm^2$ of ultraviolet irradiation (UV-crosslink). During storage the membrane was sealed with 1 ml of PBS, 0.2% SDS, per $cm^2$ of membrane in a plastic bag.
B) Standard Electrophoresis/Blotting. For standard electrophoresis, a 6% polyacrylamide gel (40 cm x 20 cm x 0.034 cm) was used with the same buffer conditions as described above. Electrophoresis was performed at a constant power of 40 watts. After the run the gel was transferred into a horizontal electroblotter (Polytech Products, Sommerville, MA) and the DNA was transferred onto a membrane for 20 min at 120 volts. After UV-crosslinking/baking, the membrane was stored in PBS, 0.2% SDS in a heat sealed plastic bag.

Equivalents

Those skilled in the art will recognize, or be able to ascertain, using no more than routine experimentation, many equivalents to the specific embodiments of the invention described herein. These and all other equivalents are intended to be encompassed by the following claims.

**Claims**

**1.** A method of detecting a nucleic acid chemiluminescense, comprising the steps of:
  a. providing a nucleic acid which is linked to a compound which can generate chemiluminescence wherein the compound is an enzyme which catalyzes a chemiluminogenic substrate to emit light;
  b. contacting the nucleic acid with a substituted 1,2-dioxetane chemiluminogenic substrate, thereby causing the compound to generate chemiluminescense; and
  c. detecting the chemiluminescense, wherein light emitted is indicative of the presence and amount of the nucleic acid.

**2.** The method of Claim 1, wherein the enzyme is selected from the group consisting of alkaline phosphatase, acid phosphatase, perioxidase, galactosidase, glucose oxidase, aryl esterase, sulfatase, urease, acetylcholinesterase, chymotrypsin, trypsin, aminopeptidase and lipase.

**3.** The method of Claim 1, wherein the chemiluminogenic substrate is a substituted 1,2-dioxetane of the formula:

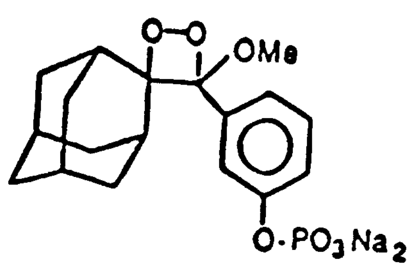

**4.** The method of Claim 1, further comprising performing step (b) in the presence of an enhancer molecule (e.g., fluorescein, coumarin and rhodamine) which is capable of amplifying the chemiluminescence, wherein the enhancer molecule is present in combination with a carrier molecule.

**5.** The method of Claim 1, wherein the nucleic acid which is linked to a compound that can generate chemiluminescence has the formula:

A-X-Sp-Y-E,
A-X-Sp-Y-$L^1$~M~$L^2$-E or
A-X-Sp-Y-$L^1$~M~E

wherein A is a nucleic acid (e.g., deoxyribonucleic acid);
X and Y are the same or different and are chemical functional groups linking the compound that can generate chemiluminescence to the nucleic acid;
Sp is a chemical spacer group that provides an appropriate linking distance between A and E;
$L^1$ and $L^2$ are the same or different and are linking units;
M is a bridging unit covalently or non-covalently linked to E; and
E is the compound that can generate chemiluminescence.

**6.** The method of Claim 5, wherein:
Sp is $(CH_2)_n$, $(CH_2)_n(C_6H_4)_m$,

$$[NH(CH_2)_nNH\overset{O}{\overset{\|}{C}}]_m,$$

$NH(CH_2)_n$, $[NH(CH_2)_nOC]_m$,

$$[NHCHR^6\overset{O}{\overset{\|}{C}}]_m,\quad [O(CH_2)_nNH\overset{O}{\overset{\|}{C}}]_m,\quad [O(CH_2)_nO\overset{O}{\overset{\|}{C}}]_m,$$

$[R^6CH(CH_2)_nNH]_m$,

$$[\overset{O}{\overset{\|}{C}}NH(CH_2)_nNH]_m,$$

$(CH_2)_nNH$,

$$[\overset{O}{\overset{\|}{C}}O(CH_2)_nNH]_m,\quad [\overset{O}{\overset{\|}{C}}R^6CHNH]_m,\quad [\overset{O}{\overset{\|}{C}}NH(CH_2)_nO]_m,$$

$$\text{or}\quad [\overset{O}{\overset{\|}{C}}O(CH_2)_nO]_m;$$

n is an integer from one to twenty and M is an integer from one to six;
X and Y are selected from the group consisting of O, S, NH, $CH_2$, NHNH, SS, OH, SH, $CO_2Z$, $CO_2H$, Br, CO, NHCO, Cl, F, HgCl, CO-$NHNH_2$, azide, CO-imidazole and N-maleimide;
Z is an alkyl, aryl or aralkyl group forming preferentially active esters;
E is an enzyme selected from the group consisting of alkaline phosphatase, acid phosphatase, peroxidase, galactosidase, glucose oxidase, luciferase, aryl esterase, sulfatase, urease, acetylcholinesterase, chymotrypsin, trypsin, aminopeptidase and lipase;
$L^1$ and $L^2$ are selected from the group consisting of biotin, enzyme, lectin specific sugars, lectins, steriods, peptides, haptens, hapten specific antibodies, biotin specific antibodies, enzyme specific antibodies, and anti-antibodies; and
M is selected from the group consisting of bi-specific antibodies, avidin/streptavidin, lectin specific sugars, lectins, biotin specific antibodies, biotin, hapten and hapten specific antibodies.

**7.** A method of sequencing DNA, comprising the steps of:

a. providing a nucleic acid primer complementary to region of DNA to be sequenced, the primer being modified for attaching a compound which can generate chemiluminescense thereto;
b. hybridizing the primer to the DNA to be sequenced;
c. extending the primer with deoxyribonucleoside triphosphates using the DNA to be sequenced as a template and terminating the extension reactions with dideoxyribonucleoside triphosphates to produce DNA molecules whose lengths depend on the point of termination from the primer;
d. separating the DNA molecules generated by each of the extension reactions according to size;
e. affixing the separated DNA molecules to a solid support;
f. attaching a compound that can generate chemiluminescense to the separated molecules at the modified primer, wherein the compound which can generate chemiluminescence is an enzyme selected from the group consisting of alkaline phosphatase, acid phosphatase, peroxidase, galactosidase, glucose oxidase, luciferase, aryl esterase, sulfatase, urease, acetylcholinesterase, chymotrypsin, trypsin, aminopeptidase and lipase;
g. causing the compound to generate chemiluminescense by contacting the enzyme with a substituted 1,2-dioxetane chemiluminogenic substrate;
h. detecting the chemiluminescence, wherein light emitted is indicative of the presence and amount of nucleic acid, to obtain a pattern of the separated DNA molecules according to size; and
i. determining nucleotide sequence based upon the pattern of separated DNA molecules.

**8.** A method of sequencing DNA, comprising the steps of:
a. hybridizing a nucleic acid primer to the DNA to be sequenced;
b. extending the primer with deoxyribonucleoside triphosphates using the DNA to be sequenced as a template;
c. terminating the extension reactions with a chain terminator which is modified for attaching a compound that can generate chemiluminescence, to thereby produce DNA molecules whose lengths depend on the point of termination from the primer;
d. separating the DNA molecules generated by each of the extension reactions according to size;
e. affixing the separated DNA molecules to a solid support;
f. attaching a compound that can generate chemiluminescence to the modified terminator, wherein the compound which can generate chemiluminescence is an enzyme selected from the group consisting of alkaline phosphatase, acid phosphatase, peroxidase, galactosidase, glucose oxidase, luciferase, aryl esterase, sulfatase, urease, acetylcholinesterase, chymotrypsin, trypsin, aminopeptidase and lipase;
g. causing the compound to generate chemiluminescence by contacting the enzyme with a substituted 1,2-dioxetane chemiluminogenic substrate;
h. detecting the chemiluminescence, wherein light emitted is indicative of the presence and amount of nucleic acid, to obtain a pattern of the separated DNA molecules according to size; and
i. determining nucleotide sequence based upon the pattern of separated DNA molecules.

**9.** A method of sequencing DNA, comprising the steps of:
a. providing DNA to be sequenced, the DNA being modified at the 5' or 3' terminus for attaching a compound which can generate chemiluminescence thereto;
b. subjecting the DNA to a set of separate chemical reactions wherein the DNA is reacted with a reagent which specifically modifies one or two of the four nucleoside bases under conditions wherein only a few bases in any DNA molecule are modified and then contacting the DNA with a reagent which cleaves the DNA at the nucleotides containing the modified base;
c. separating the cleaved DNA molecules according to size;
d. affixing the separated DNA onto a solid support;
e. attaching a compound that can generate chemiluminescence to the separated molecules at the modified 5' or 3' terminus, wherein the compound which can generate chemiluminescence is an enzyme selected from the group consisting of alkaline phosphatase, acid phosphatase, peroxidase, galactosidase, glucose oxidase, luciferase, aryl esterase, sulfatase, urease, acetylcholinesterase, chymotrypsin, trypsin, aminopeptidase and lipase;
f. causing the compound to generate chemiluminescence by contacting the enzyme with a substituted 1,2-dioxetane chemiluminogenic substrate;
g. determining the chemiluminescence, wherein the light emitted is indicative of the presence of the DNA, to obtain a pattern of the separated DNA according to size; and
h. determining nucleotide sequence based upon the pattern of DNA molecules.

10. A method of hybridizing DNA, comprising the steps of:
   a. providing nucleic acid to be probed which is affixed to a solid support;
   b. contacting the nucleic acid with a probe complementary to a region of DNA to be hybridized, the probe being linked to a compound which can generate chemiluminescence wherein the compound is an enzyme which catalyzes a chemiluminogenic substrate to emit light;
   c. hybridizing the probe to the DNA to be sequenced;
   d. contacting the nucleic acid with a substituted 1,2-dioxetane chemiluminogenic substrate, thereby causing the compound to generate chemiluminescence; and
   e. detecting the chemiluminescence wherein the light emitted is indicative of the presence and amount of the hybridized DNA.

**Patentansprüche**

1. Verfahren zum Nachweis einer Nucleinsäure-Chemilumineszenz durch
   a. Bereitstellen einer Nucleinsäure, die mit einer zur Chemilumineszenzerzeugung fähigen Verbindung verknüpft ist, wobei es sich bei der Verbindung um ein Enzym handelt, das ein chemiluminogenes Substrat zur Lichtemission katalysiert;
   b. Inberührungbringen der Nucleinsäure mit einem aus einem substituierten 1,2-Dioxetan bestehenden chemiluminogenen Substrat, um die (betreffende) Verbindung zur Erzeugung von Chemilumineszenz zu veranlassen und
   c. Nachweisen der Chemilumineszenz, wobei das emittierte Licht ein Anzeichen für das Vorhandensein und die Menge der Nucleinsäure darstellt.

2. Verfahren nach Anspruch 1, wobei das Enzym aus der Gruppe alkalische Phosphatase, Säurephosphatase, Perioxidase, Galactosidase, Glucoseoxidase, Arylesterase, Sulfatase, Urease, Acetylcholinesterase, Chymotrypsin, Trypsin, Aminopeptidase und Lipase ausgewählt ist.

3. Verfahren nach Anspruch 1, wobei das chemiluminogene Substrat aus einem substituierten 1,2-Dioxetan der Formel:

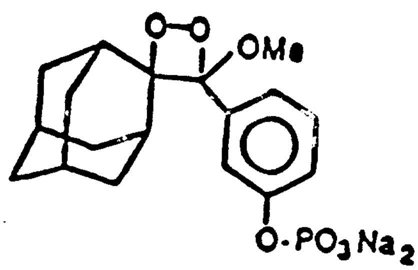

besteht.

4. Verfahren nach Anspruch 1, wobei zusätzlich die Stufe (b) in Gegenwart eines Verstärkermoleküls (beispielsweise Fluorescein, Cumarin und Rhodamin) mit der Fähigkeit zur Verstärkung der Chemilumineszenz durchgeführt wird und wobei das Verstärkermolekül in Kombination mit einem Trägermolekül vorhanden ist.

5. Verfahren nach Anspruch 1, wobei die mit einer zur Chemilumineszenzerzeugung fähigen Verbindung verknüpfte Nucleinsäure der Formel:

A-X-Sp-Y-E,
A-X-Sp-Y-$L^1$~M~$L^2$-E oder
A-X-Sp-Y-$L^1$~M~E

worin bedeuten:
A eine Nucleinsäure (beispielsweise Desxoxyribonucleinsäure);
X und Y, die gleich oder verschieden sein können, chemische funktionelle Gruppen, die die zur Chemilumineszenzerzeugung fähige Verbindung mit der Nucleinsäure verknüpfen;

Sp eine chemische Abstandsgruppe, die zwischen A und E für eine geeignete Verknüpfungsdistanz sorgt;
$L^1$ und $L^2$, die gleich oder verschieden sein können, verknüpfende Einheiten;
M eine mit E kovalent oder nicht-kovalent verknüpfte Brückeneinheit und
E die zur Chemilumineszenzerzeugung fähige Verbindung entspricht.

6. Verfahren nach Anspruch 5, worin bedeuten:
Sp $(CH_2)_n$, $(CH_2)_n(C_6H_4)_m$,

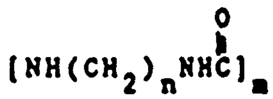,

$NH(CH_2)_n$, $[NH(CH_2)_nOC]_m$,

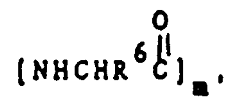, 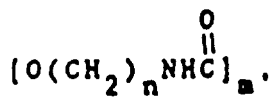, 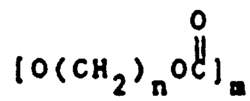,

$[R^6CH(CH_2)_nNH]_m$,

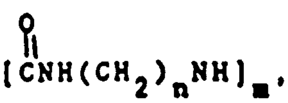,

$(CH_2)_nNH$,

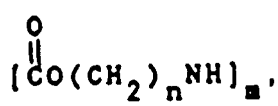, 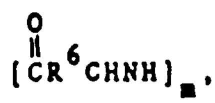, 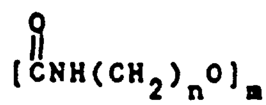,

oder 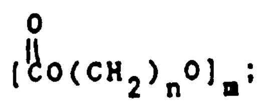;

mit n gleich einer ganzen Zahl von 1 bis 20 und m gleich einer ganzen Zahl von 1 bis 6;
X und Y O, S, NH, $CH_2$, NHNH, SS, OH, SH, $CO_2Z$, $CO_2H$, Br, CO, NHCO, Cl, F, HgCl, CO-$NHNH_2$, Azid, CO-Imidazol oder N-Maleimid;
Z eine Alkyl-, Aryl- oder Aralkylgruppe, die vorzugsweise aktive Ester bildet;
E ein Enzym aus der Gruppe alkalische Phosphatase, Säurephosphatase, Peroxidase, Galactosidase, Glucoseoxidase, Luciferase, Arylesterase, Sulfatase, Urease, Acetylcholinesterase, Chymotrypsin, Trypsin, Aminopeptidase und Lipase;
$L^1$ und $L^2$ jeweils eine Komponente aus der Gruppe Biotin, Enzym, lectinspezifische Zucker, Lectine, Steroide, Peptide, Haptene, haptenspezifische Antikörper, biotinspezifische Antikörper, enzymspezifische Antikörper und Anti-Antikörper und
M eine Komponente aus der Gruppe bispezifische Antikörper, Avidin/Streptavidin, lectinspezifische Zucker, Lectine, biotinspezifische Antikörper, Biotin, Hapten und haptenspezifische Antikörper.

7. Verfahren zur Sequenzierung von DNA durch:
a. Bereitstellen eines Nucleinsäureprimers, der zu dem zu sequenzierenden DNA-Bereich komplementär ist, wobei der Primer zum Anlagern einer zur Chemilumineszenzerzeugung fähigen Verbindung modifiziert ist;
b. Hybridisieren des Primers an die zu sequenzierende DNA;
c. Verlängern des Primers mit Desoxyribonucleosidtriphosphaten unter Verwendung der zu sequenzierenden DNA als einer Matrize und Beendigen der Verlängerungsreaktionen mit Didesoxyribonucleosidtriphosphaten zur Herstellung von DNA-Molekülen, deren Längen vom Terminationspunkt von dem Primer abhängen;
d. Auftrennen der durch die einzelnen Verlängerungsreaktionen erzeugten DNA-Moleküle entsprechend ihrer Größe;
e. Befestigen der abgetrennten DNA-Moleküle an einem festen Träger;
f. Anlagern einer zur Chemilumineszenzerzeugung fähigen Verbindung an die abgetrennten Moleküle am modifizierten Primer, wobei es sich bei der zur Chemilumineszenzerzeugung fähigen Verbindung um ein Enzym aus der Gruppe alkalische Phosphatase, Säurephosphatase, Peroxidase, Galactosidase, Glucoseoxidase, Luciferase, Arylesterase, Sulfatase, Urease, Acetylcholinesterase, Chymotrypsin, Trypsin, Aminopeptidase und Lipase handelt;
g. Veranlassen der Verbindung zur Chemilumineszenzerzeugung durch Inberührungbringen des Enzyms mit einem aus einem substituierten 1,2-Dioxetan bestehenden chemiluminogenen Substrat;
h. Nachweisen der Chemilumineszenz, wobei emittiertes Licht ein Anzeichen für das Vorhandensein und die Menge an Nucleinsäure darstellt, um ein Muster der getrennten DNA-Moleküle entsprechend ihrer Größe zu gewinnen, und
i. Bestimmen einer Nucleotidsequenz auf der Basis des Musters der aufgetrennten DNA-Moleküle.

**8.** Verfahren zur Sequenzierung von DNA durch:
a. Hybridisieren eines Nucleinsäureprimers an die zu sequenzierende DNA;
b. Verlängern des Primers mit Desoxyribonucleosidtriphosphaten unter Verwendung der zu sequenzierenden DNA als einer Matrize;
c. Beenden der Verlängerungsreaktionen mit einem Kettenabbruchmittel, das zur Anlagerung einer zur Chemilumineszenzerzeugung fähigen Verbindung modifiziert ist, um DNA-Moleküle herzustellen, deren Längen vom Terminationspunkt vom Primer abhängen;
d. Auftrennen der durch die einzelnen Verlängerungsreaktionen erzeugten DNA-Moleküle entsprechend ihrer Größe;
e. Befestigen der abgetrennten DNA-Moleküle an einem festen Träger;
f. Anlagern einer zur Chemilumineszenzerzeugung fähigen Verbindung an den modifizierten Terminator, wobei es sich bei der zur Chemilumineszenzerzeugung fähigen Verbindung um ein Enzym aus der Gruppe alkalische Phosphatase, Säurephosphatase, Peroxidase, Galactosidase, Glucoseoxidase, Luciferase, Arylesterase, Sulfatase, Urease, Acetylcholinesterase, Chymotrypsin, Trypsin, Aminopeptidase und Lipase handelt;
g. Veranlassen der Verbindung zur Chemilumineszenzerzeugung durch Inberührungbringen des Enzyms mit einem aus einem substituierten 1,2-Dioxetan bestehenden chemiluminogenen Substrat;
h. Nachweisen der Chemilumineszenz, wobei emittiertes Licht ein Anzeichen für das Vorhandensein und die Menge an Nucleinsäure darstellt, um ein Muster der getrennten DNA-Moleküle entsprechend ihrer Größe zu gewinnen, und
i. Bestimmen einer Nucleotidsequenz auf der Basis des Musters der aufgetrennten DNA-Moleküle.

**9.** Verfahren zur Sequenzierung von DNA durch:
a. Bereitstellen einer zu sequenzierenden DNA, die am 5'- oder 3'-Ende zur Anlagerung einer zur Chemilumineszenzerzeugung fähigen Verbindung modifiziert ist;
b. Unterwerfen der DNA einer Reihe getrennter chemischer Reaktionen, wobei die DNA mit einem Reagens, das spezifisch eine oder zwei der vier Nucleosidbasen unter Bedingungen, unter denen lediglich einige wenige Basen in jedem DNA-Molekül modifiziert werden, modifiziert zur Umsetzung gebracht und danach die DNA mit einem Reagens, das die DNA an den die modifizierte Base enthaltenden Nucleotiden spaltet, in Berührung gebracht wird;
c. Auftrennen der gespaltenen DNA-Moleküle entsprechend ihrer Größe;
d. Befestigen der abgetrennten DNA auf einem festen Träger;
e. Anlagern einer zur Chemilumineszenzerzeugung fähigen Verbindung an den aufgetrennten Molekülen am modifizierten 5'- oder 3'-Ende, wobei die zur Chemilumineszenzerzeugung fähige Verbindung aus einem Enzym aus der Gruppe alkalische Phosphatase, Säurephosphatase, Peroxidase, Galactosidase, Glucoseoxidase, Luciferase, Arylesterase, Sulfatase, Urease, Acetylcholinesterase, Chymotrpysin, Trypsin, Aminopeptidase und Lipase ausgewählt ist;
f. Veranlassen der Verbindung zur Chemilumineszenzerzeugung durch Inberührungbringen des Enzyms mit einem aus einem substituierten 1,2-Dioxetan bestehenden chemiluminogenen Substrat;
g. Nachweisen der Chemilumineszenz, wobei emittiertes Licht ein Anzeichen für das Vorhandensein und die Menge an DNA darstellt, um ein Muster der abgetrennten DNA entsprechend ihrer Größe zu

gewinnen, und

h. Bestimmen einer Nucleotidsequenz auf der Basis des Musters der aufgetrennten DNA-Moleküle.

**10.** Verfahren zum Hybridisieren von DNA durch:

a. Bereitstellen einer zu sondenden Nucleinsäure, die an einem festen Träger befestigt ist;

b. Inberührungbringen der Nucleinsäure mit einer Sonde, die zu einem zu hybridisierenden DNA-Bereich komplementär ist, wobei die Sonde mit einer zur Chemilumineszenzerzeugung fähigen Verbindung verknüpft ist und die (betreffende) Verbindung aus einem ein chemiluminogenes Substrat zur Lichtemission katalysierenden Enzym besteht;

c. Hybridisieren der Sonde an die zu sequenzierende DNA;

d. Inberührungbringen der Nucleinsäure mit einem aus einem substituierten 1,2-Dioxetan bestehenden chemiluminogenen Substrat, um die (betreffende) Verbindung zur Chemilumineszenzerzeugung zu veranlassen, und

e. Nachweisen der Chemilumineszenz, wobei das emittierte Licht ein Anzeichen für das Vorhandensein und die Menge an der hybridisierten DNA darstellt.

## Revendications

**1.** Méthode de détection de la chimioluminescence d'un acide nucléique, comprenant les étapes :

a. de préparation d'un acide nucléique qui est lié à un composé qui peut engendrer une chimioluminescence, le composé étant un enzyme qui catalyse l'émission de lumière par un substrat chimioluminogène ;

b. de mise en contact de l'acide nucléique avec un substrat chimioluminogène consistant en un 1,2-dioxétanne substitué, ce qui amène le composé à engendrer une chimioluminescence ; et

c. de détection de la chimioluminescence, dans laquelle la lumière émise constitue une indication de la présence et de la quantité de l'acide nucléique.

**2.** Méthode suivant la revendication 1, dans laquelle l'enzyme est choisi dans le groupe consistant en une phosphatase alcaline, une phosphatase acide, une peroxydase, une galactosidase, une glucose-oxydase, une aryl-estérase, une sulfatase, une uréase, une acétylcholinestérase, une chymotrypsine, une trypsine, une amino-peptidase et une lipase.

**3.** Méthode suivant la revendication 1, dans laquelle le substrat chimioluminogène est un 1,2-dioxétanne substitué de formule

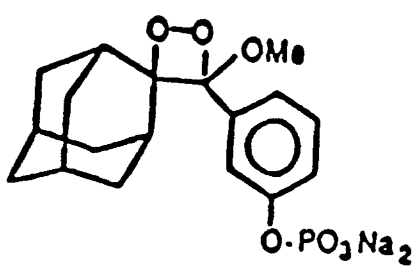

**4.** Méthode suivant la revendication 1, comprenant en outre la mise en oeuvre de l'étape (b) en présence d'une molécule amplificatrice (par exemple la fluorescéine, la coumarine et la rhodamine) qui est capable d'amplifier la chimioluminescence, dans laquelle la molécule amplificatrice est présente en association avec une molécule de support.

**5.** Méthode suivant la revendication 1, dans laquelle l'acide nucléique qui est lié à un composé qui peut engendrer une chimioluminescence répond à la formule

A-X-Sp-Y-E.

A-X-Sp-Y-$L^1$-M-$L^2$-E ou

A-X-Sp-Y-$L^1$-M-E

dans laquelle A représente un acide nucléique (par exemple un acide désoxyribonucléique) ;

X et Y sont identiques ou différents et représentent des groupes fonctionnels chimiques liant le composé qui peut engendrer une chimioluminescence à l'acide nucléique ;

Sp représente un groupe chimique d'espacement qui engendre une distance de liaison appropriée entre A et E ;

$L^1$ et $L^2$ sont identiques ou différents et représentent des motifs de liaison ;

M est une unité de pontage liée de manière covalente ou non covalente à E ; et

E est le composé qui peut engendrer une chimioluminescence.

**6.** Méthode suivant la revendication 5, dans laquelle :

Sp représente un groupe $(CH_2)_n$, $(CH_2)_n(C_6H_4)_m$,

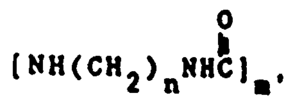

$NH(CH_2)_n$, $[NH(CH_2)_nOC]_m$,

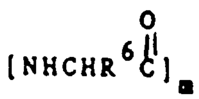 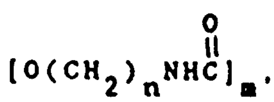 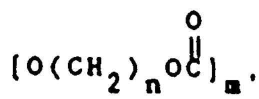

$[R^6CH(CH_2)_nNH]_m$,

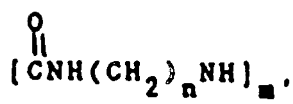

$(CH_2)_nNH$,

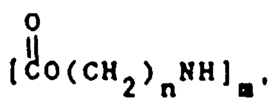 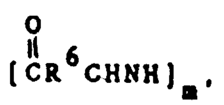 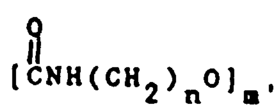

ou 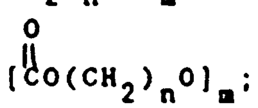

n est un nombre entier de un à vingt et M est un nombre entier de un à six ;

X et Y sont choisis entre les groupes O, S, NH, $CH_2$, NHNH, SS, OH, SH, $CO_2Z$, $CO_2H$, Br, CO, NHCO, Cl, F, HgCl, CO-$NHNH_2$, azoture, CO-imidazole et N-maléimido ;

Z représente un groupe alkyle, aryle ou aralkyle formant préférentiellement des esters actifs ;

E représente un enzyme choisi dans le groupe consistant en une phosphatase alcaline, une phosphatase acide, une peroxydase, une galactosidase, une glucose-oxydase, une luciférase, une aryl-estérase, une sulfatase, une uréase, une acétylcholinestérase, une chymotrypsine, une trypsine, une aminopeptidase et une lipase ;

$L^1$ et $L^2$ sont choisis dans le groupe consistant en la biotine, un enzyme, des sucres spécifiques des lectines, des lectines, des stéroides, des peptides, des haptènes, des anticorps spécifiques d'haptènes, des anticorps spécifiques de la biotine, des anticorps spécifiques d'enzymes et des anti-anticorps ; et

M est choisi dans le groupe consistant en anticorps bi-spécifiques, l'association avidine/streptavidine, des sucres spécifiques de lectines, des lectines, des anticorps spécifiques de la biotine, la biotine, un haptène et des anticorps spécifiques d'haptènes.

**7.** Méthode de séquençage d'ADN, comprenant les étapes :

a. de préparation d'une amorce d'acide nucléique complémentaire de la région d'ADN à séquencer, l'amorce étant modifiée pour la fixation à cette amorce d'un composé qui peut engendrer une chimioluminescence ;
b. d'hybridation de l'amorce à l'ADN à séquencer ;
c. d'extension de l'amorce avec des désoxyribonucléoside-triphosphates au moyen de l'ADN à séquencer comme matrice et de terminaison des réactions d'extension avec des didésoxyribonucléoside-triphosphates pour produire des molécules d'ADN dont les longueurs dépendent du point de terminaison à partir de l'amorce ;
d. de séparation des molécules d'ADN engendrées par chacune des réactions d'extension, en fonction des dimensions ;
e. de fixation des molécules d'ADN séparées à un support solide ;
f. de fixation d'un composé qui peut engendrer une chimioluminescence aux molécules séparées au niveau de l'amorce modifiée, le composé qui peut engendrer une chimioluminescence étant un enzyme choisi dans le groupe consistant en une phosphatase alcaline, une phosphatase acide, une peroxydase, une galactosidase, une glucose-oxydase, une luciférase, une arylestérase, une sulfatase, une uréase, une acétylcholinestérase, une chymotrypsine, une trypsine, une aminopeptidase et une lipase ;
g. de réalisation des conditions amenant le composé à engendrer une chimioluminescence par mise en contact de l'enzyme avec un substrat chimioluminogène consistant en un 1,2-dioxétanne substitué ;
h. de détection de la chimioluminescence, dans laquelle la lumière émise constitue une indication de la présence et de la quantité d'acide nucléique, pour obtenir un motif des molécules d'ADN séparées en fonction des dimensions ; et
i. de détermination de la séquence de nucléotides sur la base du motif des molécules d'ADN séparées.

**8.** Méthode de séquençage d'ADN, comprenant les étapes :
a. d'hybridation d'une amorce d'acide nucléique à l'ADN à séquencer ;
b. d'extension de l'amorce avec des désoxyribonucléoside-triphosphates au moyen de l'ADN à séquencer comme matrice ;
c. de terminaison des réactions d'extension avec un terminateur de chaîne qui est modifié pour la fixation d'un composé qui peut engendrer une chimioluminescence, ce qui permet de produire des molécules d'ADN dont les longueurs dépendent du point de terminaison à partir de l'amorce ;
d. de séparation des molécules d'ADN engendrées par chacune des réactions d'extension, en fonction des dimensions ;
e. de fixation des molécules d'ADN séparées à un support solide ;
f. de fixation d'un composé qui peut engendrer une chimioluminescence au terminateur modifié, le composé qui peut engendrer une chimioluminescence étant un enzyme choisi dans le groupe consistant en une phosphatase alcaline, une phosphatase acide, une peroxydase, une galactosidase, une glucose-oxydase, une luciférase, une arylestérase, une sulfatase, une uréase, une acétylcholinestérase, une chymotrypsine, une trypsine, une aminopeptidase et une lipase ;
g. de réalisation des conditions amenant le composé à engendrer une chimioluminescence par mise en contact de l'enzyme avec un substrat chimioluminogène consistant en un 1,2-dioxétanne substitué ;
h. de détection de la chimioluminescence, la lumière émise constituant une indication de la présence et de la quantité de l'acide nucléique, pour obtenir un motif des molécules d'ADN séparées, en fonction des dimensions ; et
i. de détermination de la séquence de nucléotides sur la base du motif des molécules d'ADN séparées.

**9.** Méthode de séquençage d'ADN, comprenant les étapes :
a. de préparation d'un ADN à séquencer, l'ADN étant modifié à l'extrémité 5' ou 3' pour la fixation à cet ADN d'un composé qui peut engendrer une chimioluminescence ;
b. de conduite d'une série de réactions chimiques distinctes dans lesquelles l'ADN est amené à réagir avec un réactif qui modifie spécifiquement une ou deux des quatre bases de nucléosides dans des conditions dans lesquelles seulement quelques bases dans une quelconque molécule d'ADN sont modifiées, puis de mise en contact de l'ADN avec un réactif qui clive l'ADN au niveau des nucléotides contenant la base modifiée ;

c. de séparation des molécules d'ADN clivées, en fonction des dimensions ;
d. de fixation de l'ADN séparé sur un support solide ;
e. de fixation d'un composé qui peut engendrer une chimioluminescence aux molécules séparées, au niveau de l'extrémité 5' ou 3' modifiée, le composé qui peut engendrer une chimioluminescence étant un enzyme choisi dans le groupe consistant en une phosphatase alcaline, une phosphatase acide, une peroxydase, une galactosidase, une glucose-oxydase, une luciférase, une aryl-estérase, une sulfatase, une uréase, une acétylcholinestérase, une chymotrypsine, une trypsine, une amino-peptidase et une lipase ;
f. de réalisation des conditions amenant le composé à engendrer une chimioluminescence par mise en contact de l'enzyme avec un substrat chimioluminogène consistant en un 1,2-dioxétanne substitué ;
g. de détermination de la chimioluminescence, la lumière émise constituant une indication de la présence de l'ADN, pour obtenir un motif de l'ADN séparé, en fonction des dimensions ; et
h. de détermination de la séquence de nucléotides sur la base du motif des molécules d'ADN.

**10.** Méthode d'hybridation d'ADN, comprenant les étapes :
a. de préparation d'un acide nucléique à sonder, qui est fixé à un support solide ;
b. de mise en contact de l'acide nucléique avec une sonde complémentaire d'une région d'ADN à hybrider, la sonde étant liée à un composé qui peut engendrer une chimioluminescence, le composé étant un enzyme qui catalyse l'émission de lumière par un substrat chimioluminogène ;
c. d'hybridation de la sonde à l'ADN à séquencer ;
d. de mise en contact de l'acide nucléique avec un substrat chimioluminogène consistant en un 1,2-dioxétanne substitué, ce qui amène le composé à engendrer une chimioluminescence, et
e. de détection de la chimioluminescence, la lumière émise constituant une indication de la présence et de la quantité de l'ADN hybridé.

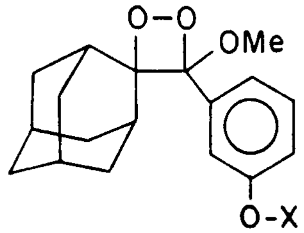

FIG. Ia

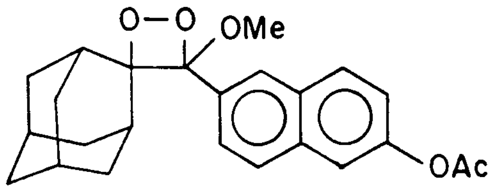

FIG. Ib

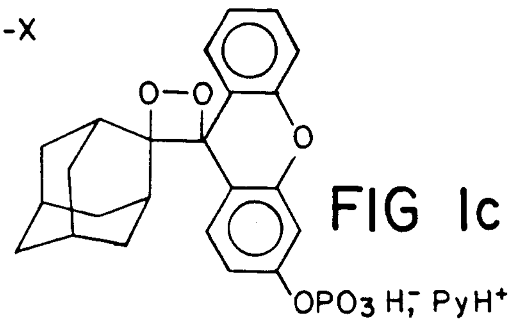

FIG Ic

| Substituent X | Enzyme |
|---|---|
| $-PO_3Na_2$ | Alkaline Phosphatase |
| -COMe | Aryl Esterase |
| $-SO_3Na$ | Sulfatase |
| -H | Urease/Urea |
| β-Galactoside | β-Galactosidase |

LEGEND PERTAINS TO FIG. Ia, FIG. Ib AND FIG. Ic

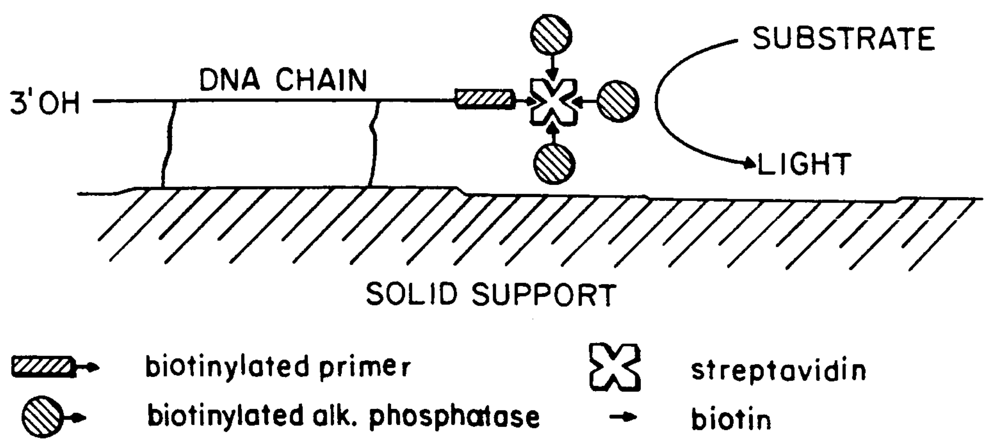


FIG. 2

FIG.3a

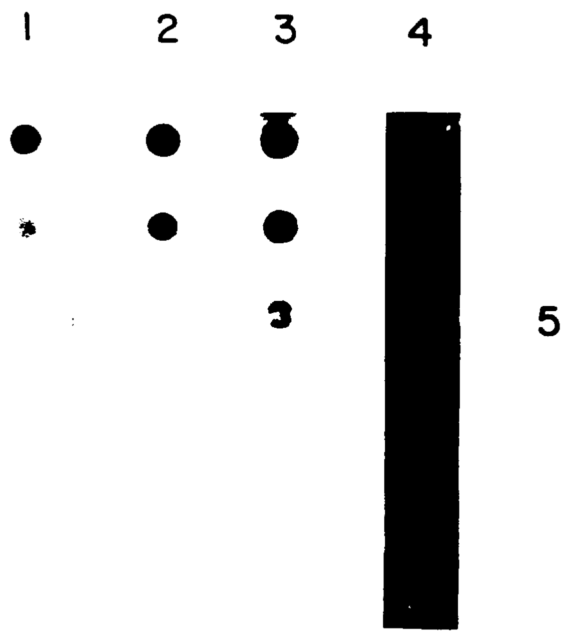

FIG.3b

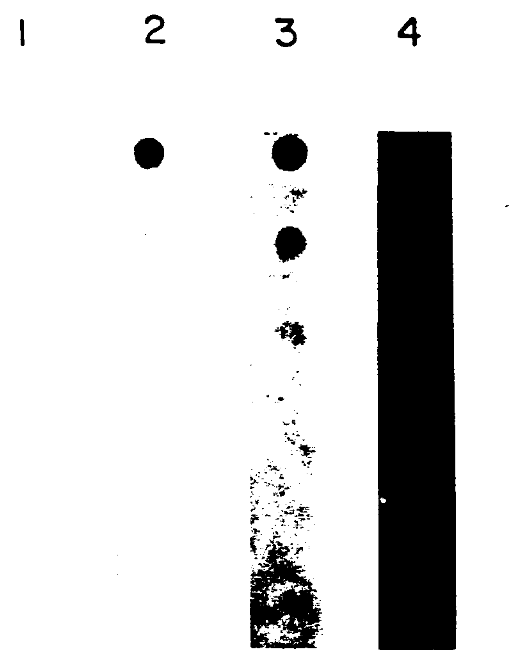

FIG.3c

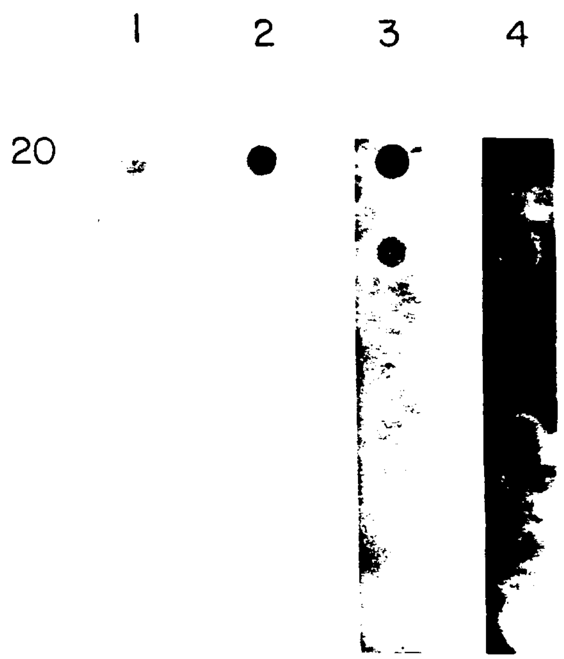

FIG.3d

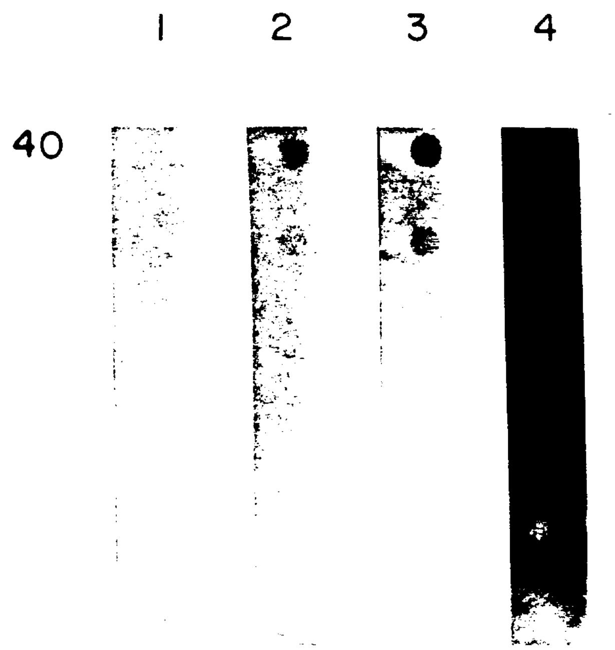

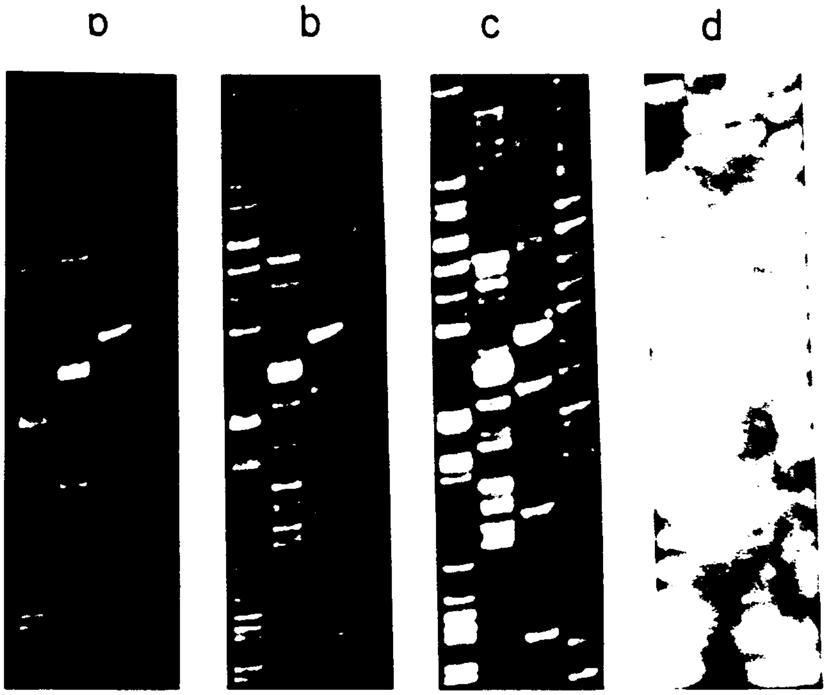


FIG.4

FIG.5a FIG.5b